(19) Europäisches Patentamt
European Patent Office
Office européen des brevets

(11) Publication number: 0 320 942 A2

# (12) EUROPEAN PATENT APPLICATION

(21) Application number: 88121017.3

(22) Date of filing: 15.12.88

(51) Int. Cl.4: C07H 15/04 , A61K 39/102

(30) Priority: 18.12.87 US 134664

(43) Date of publication of application:
21.06.89 Bulletin 89/25

(84) Designated Contracting States:
AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Just, George Erich
134 Rue Principale
Ville De L'Ile Cadieux Quebec, J7V5V5(CA)
Inventor: Upeslacis, Janis
3 Keim Drive
Pomona New York 10970(US)

(74) Representative: Wächtershäuser, Günter, Dr.
Tal 29
D-8000 München 2(DE)

## (54) Novel polysaccharides novel macromolecular conjugates of the polysaccharides.

(57) Synthesis of oligomeric subunits of the poly(ribosyl-ribitol-phosphate) capsular polysaccharide found on Haemophilus influenzae type b bacteria. The covalent attachment of these compounds to immunogenic proteins without using spacer elements.

EP 0 320 942 A2

# NOVEL POLYSACCHARIDES AND NOVEL MACROMOLECULAR CONJUGATES OF THE POLYSACCHARIDES

## BACKGROUND OF THE INVENTION

This invention teaches the synthesis of oligomeric subunits of the poly-(ribosyl-ribitol-phosphate) capsular polysaccharide found on Haemophilus influenzae type b bacteria. This invention describes the covalent attachment of these compounds to immunogenic proteins without the necessity of using spacer elements. Further, the invention contemplates the use of these complexes as vaccines to prevent Haemophilus bacterial infections in mammals.

Bacterial meningitis due to Haemophilus influenzae remains one of the few serious and life-threatening diseases of childhood that has not been brought under effective control. The use of antibiotics has reduced the mortality rate of the disease from more than 90% to between 5 and 10%. However, 30 to 50% of the survivors suffer permanent neurological sequelae such as blindness, deafness, mental retardation and hydrocephalus. At present, Haemophilus influenzae type b (HI-b) is the leading cause of endemic meningitis in the U.S., occurring mainly among children between 3 months and 4 years of age. [M. Solotorovsky, M. Lynn, CRC Crit. Rev. Microbiol. 6, 1 (1978)].

The organism, a Gram-negative coccobacillus, was first isolated by Pfeiffer from the nasopharynx of persons diagnosed as having influenza during the 1889-1892 pandemic.

Pittmann [M.J. Exp. Med. 58, 471 (1931); ibid 58, 683 (1933) noted that strains of Haemophilus influenzae from cerebrospinal fluid, grown on a transparent medium, produced two types of colonies described as smooth (S) and rough (R) by their appearances. The S and R strains had different pathogenic and antigenic properties. To explain that, Pittman postulated the presence of a capsule on S organisms which enhanced the virulence of these organisms. Six antigenic types of capsules were then demonstrated and designated with the letters a through f.

The capsular materials of types a, b, c, d, and f have been identified as polysaccharides with all except type d containing phosphorous [C.F. MacPherson, et.al., J. Immunol. 52, 207 (1946)]. The capsular substance of type b, the most frequently encountered type from haemophilus meningitis, contained ribose. In 1953, Zamenhof et al. [J. Biol. Chem. 203, 695 (1953)] postulated that the type-specific substance of HI-b was composed of polyribosephosphate chains with 3:5-phosphate diester linkages between the ribose moieties. Two of these chains were linked together by 1:1-glycosidic bonds. Further publications [E. Rosenberg, S. Zamenhof, J. Biol. Chem. 236, 2845 (1961); ibid 237, 1040 (1961)] established the double ribose unit as β-D-ribf-β-D-ribf and proposed the structure I.

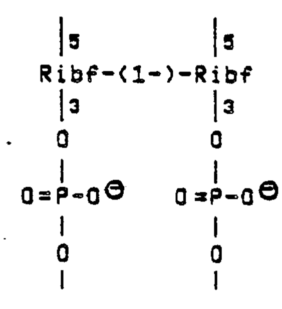

I

Based on a later finding of ribitol as a degradation product from HI-b, the structure I proposed by Zamenhof et al. became suspect. In order to resolve this matter, a reinvestigation of the structure was undertaken by Crisel et. al. [J. Biol. CHem. 250, 4926 (1975)] who confirmed the presence of ribitol in HI-b, and found equimolar proportions of ribose, ribitol and phosphate. Periodate oxidation studies, paper chromatography of acidic and alkaline hydrolysates, and $^{13}C$ NMR spectral data indicated the structure of HI-b to be a polyribofuranosylribitolphosphate (PRP) polymer II.

-3)-Ribf-(1-1)-Ribitol-(5-$OPO_2O$-

Further structural studies by Branefors-Helander et. al. [Acta Chem. Scand. B, 30, 276 (1976)] demonstrated that the ribitol moiety has the D-configuration. The phosphate, linked to the other primary position in the ribitol moiety, is consequently linked to 0-5 in D-ribitol. This is in agreement with the postulated biosynthetic route [J. Baddiley, Acc. Chem. Res. 3, 98 (1970)] in which ribitol phosphate most probably derives from cytidine diphosphate ribitol, in which D-ribitol is phosphorylated at 0-5. Based on these findings, a complete stereochemical structure of HI-b was proposed as III.

3)-β-D-Ribf-(1-1)-D-Ribitol-(5-$OPO_2O$-

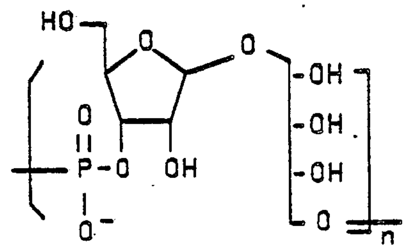

III

Protection against haemophilus meningitis is associated with humoral immunity. The role of antibody-mediated immunity was established by passive protection of experi mental animals and successful serotheraphy in humans. Since most serious diseases caused by Haemophilus influenzae are associated with type b organisms, the type-specific capsular material was a logical choice for use as an antigen capable of inducing a protective immune response. A PRP used as a vaccine was prepared by alcohol precipitation from aqueous extracts of agar-grown organisms. This material had a molecular weight of about 200,000 daltons as determined by gel filtration [L. Rodrigues, et. al., J. Immunol. 107, 1071 (1971)]. Immunization of adult humans with doses of PRP, ranging from 0.1 to 50 ug, induced an increase in titers of anti-PRP antibodies [G. Peters, et. al., "Haemophilus Influenzae," S.H.W. Sill, D.T. Karzon eds., Vanderbilt University, Press, Nashville, 281 (1972)]. In contrast to adults, titers induced by injection of 10 or 50 ug PRP in children decreased after two months. Antibody response to PRP was found to be age-dependent among children.

Although PRP is effective in increasing antibody titers in adults with pre-existing antibody levels, the usefulness of PRP as an effective vaccine is dependent on the response of disease-susceptible young children. It is known now that children under two years of age do not develop a significant immune response [D.H. Smith, Hosp. Pract. 11, 71 (1976)]. Since 70% of the cases of haemophilus meningitis occur in this age group, PRP does not appear to be an effective immunogen. It has been found that anti-PRP antibodies can only be elicited in animals when the encapsulated organism, rather than PRP itself, is injected. Thus, PRP may be a hapten, requiring the participation of a carrier.

An alternative approach toward making a more effective vaccine has been to make PRP, a poor immunogen, into a better immunogen [P. Anderson, et. al., J. Infect. Dis. 152, 634 (1985); D.M. Granoff, K.L. Cates, J. Pediatr., 107, 330 (1985)]. This has been achieved in part by conjugating purified PRP or its acid hydrolysis fragments to proteins such as diphtheria toxoid [L. Gordon, Eur. Pat. Appln. #98,581 (1984); J. Eskola, et. al., Lancet 1985, I, No. 8439, 1184] or $CRM_{197}$ [P. Anderson, Infect. Immunity 39, 233 (1983)); ibid. J. Clin. Invest. 76, 52 (1985); ibid. J. Pediatr., 107, 346 (1985)]. These vaccines have elicited protective antibody responses against H. influenzae in children as young as six months of age. Technical problems limit the use of either type of vaccine. Since native PRP must be isolated from a pathogen, issues of reproducibility, variable potency, and especially safety must be addressed, both as they apply to clinical use of PRP as well as during its manufacturing.

The entire PRP molecule is not necessary to elicit a protective response. Smaller acid hydrolysis

fragments, when covalently attached to immunogenic proteins, are as effective as the native material in animal studies [P. Anderson, Infect. Immunity 39, 233 (1983)]. The possibility exists that just a few PRP oligomeric units, accessible through chemical synthesis, would suffice. Two groups have published their attempts to prepare the minimum PRP antigen. Garegg et. al. have synthesized 1-O-β-D-ribofuranosyl-D-ribitol [Carbohyd. Res. 58, 219 (1977)] and its corresponding 5-phosphate ester [Carbohyd. Res. 86, 293 (1980)]. The latter, containing all three structural units of monomoeric PRP was not immunologically active. A phosphodiester isostere containing functionality for conjugation to a protein carrier, 4-aminophenyl β-D-ribofuranoside 3-(D-ribit-5-ylphosphate) has been synthesized as well [P.J. Garegg et. al. Carbohyd. Res. 150, 285 (1986)].

More recently, Hoogerhout, et. al. [Tet. Lett. 28, 1553 (1987)] reported their preparation of dimeric and trimeric PRP derivatives (IV, n = 2 or 3) containing an N-(3-oxo-1-propyl) glycinamide spacer for attachment of the materials to proteins. The trimer is reported to be as good or better than native PRP in eliciting an immunogenic response when conjugated to tetanus toxoid. However, this last approach suffers from the necessity of incorporating a spacer between the synthetic PRP and the carrier protein. Any antibody response against the spacer may lead to an unnecessary and even toxic immunological side-reaction.

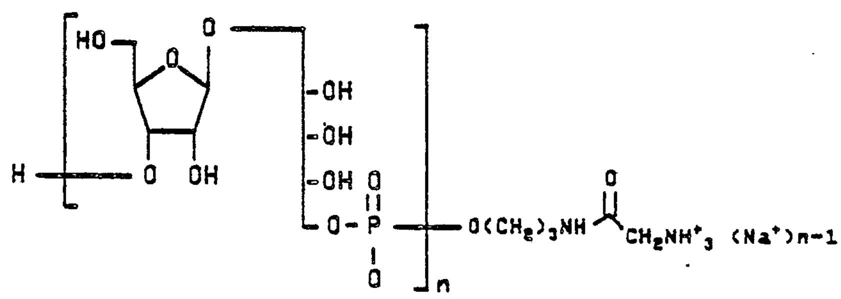

IV

Large antigens, such as native PRP itself, do not always require conjugation to immunogenic proteins, but small antigens do. A variety of methods are available to accomplish this. The most common method involves Schiff's base formation between an amine on one conjugation partner and an aldehyde (such as the reducing end of a carbohydrate) on the other, followed by reduction.

A second approach [R. Schneersdon et. al., J. Exp. Med. 152, 361 (1980)] involves coupling the protein to one end of a bifunctional molecule such as adipic acid dihydrazide. This is accomplished with carbodiimide and presumably forms a diacylated hydrazine with the pendant glutamic or aspartic carboxyl groups. This product is then reacted with a cyanogen bromide activated polysaccharide, presumably forming an O-alkyl-4-acylated isosemicarbazide. It has been suggested that the term "monogeneric spacer" conjugate be used to describe this type of product.

A third approach [S. Marburg, et. al., J. Am. Chem. Soc. 108, 5282 (1986)] which results in a "bigeneric spacer" product involves functionalizing each reaction partner with a bifunctional "spacer" molecule. Each is chosen so that the remaining valency can react with that of its opposite partner to form a stable covalent bond. Use of a synthetic version of PRP rather than purified native PRP itself for preparing protein conjugates offers several advantages. Synthetic material can be specifically radiolabelled, facilitating purification of conjugates as well as permitting measurement of coupling efficiency and carbohydrate-to-protein ratios. A good synthetic strategy also avoids the complicated multistep procedures involved with using mono- or bigeneric spacers, since the spacer itself can be built into the synthetic material and varied as necessary for maximum conjugation efficiency. In addition, whereas native PRP is generally crosslinked to a protein carrier at random points along its backbone, synthetic PRP can be conjugated through a single point.

Any conjugation procedure which requires the use of a spacer unit between the carrier protein and the antigen brings with it the risk of eliciting an immune response in a host against the spacer. This possibility, since it can lead to unnecessary immunological side-effects, argues against using any spacing at all. Therefore, it was felt that an ideal spacer would be one derived from a representative part of the polysaccharide molecule itself.

## SUMMARY OF THE INVENTION

This invention is novel polysaccharides and novel macromolecular conjugates of the polysaccharides.

This invention teaches the synthesis of molecules containing the repeating units ribitol-phospho-ribose as fragments of the Haemophilus influenzae type b capsular polysaccharide. More particularly, it is concerned with the synthesis of novel polysaccharides of Formula I.

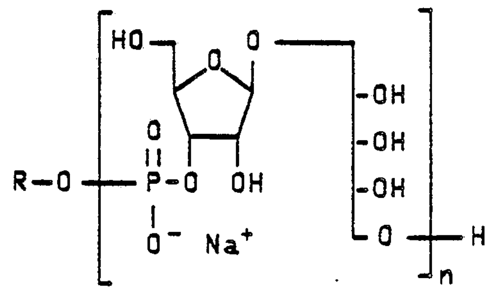

Formula I

wherein n is an integer of from 2 to 20, inclusive, and R is a spacer fragment defined by Formula II.

$R'-(CHOH)_m-CH_2-$ Formula II

wherein m is an integer of from 0 to 3, inclusive, and R′ is a functional group such as CHO, COY, $CH_2X$, $CH_2NH_2$, or $CH_2SH$, wherein Y is a carboxyl activating functionality such as halo, N-hydroxysuccinimido, hydrazino, and the like, and X is an amino-reactive group such halo, methanesulfonyl, trifluoromethanesulfonyl, toluenesulfonyl, and the like.

This invention also teaches the preparation of macromolecular conjugates of the compounds of Formula I, wherein R, n and m are as hereinbefore defined and R′ is

macromolecule $-S-S-CH_2-$

macromolecule

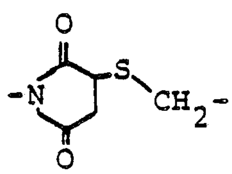

macromolecule -NHCO-

macromolecule $-NHCH_2-$

macromolecule -N=CH-

macromolecule $-CONHCH_2-$

macromolecule $-CH_2NHNHCO-$

macromolecule -CH=NNHCO-

macromolecule $-CH_2NHNHCO-$

wherein the macromolecule is defined as any one of a number of immunogenic carriers, such as proteins like tetanus toxin or toxoid, diphtheria toxin or toxoid, KLH, outer membrane porin protein, and the like, which are capable of inducing a T-cell dependent response to the synthetic polysaccharide.

This invention furthermore relates to a method for inducing a T-cell dependent immune response against Haemophilus influenzae type b by vaccinating mammals with an effective amount of the above macromolecular conjugates, alone or in combinations with other vaccine regimens.

This invention additionally relates to the use of the compounds of Formula I as diagnostic reagents for determining the pre-existance of neutralizing antibodies against Haemophilus influenzae in the sera of mammals.

This invention further relates to a pharmaceutical composition which comprises an effective amount of the above compounds as recited, in association with pharmaceutically acceptable carriers and adjuvants.

It is contemplated that a solid phase synthesis of the polysaccharides of this invention will produce layer oligomeric units.

Finally, this invention relates to a process for preparation of compounds and immunological conjugates as recited above.

## DETAILED DESCRIPTION OF THE INVENTION

The compounds of this invention can be prepared according to the following reaction scheme.

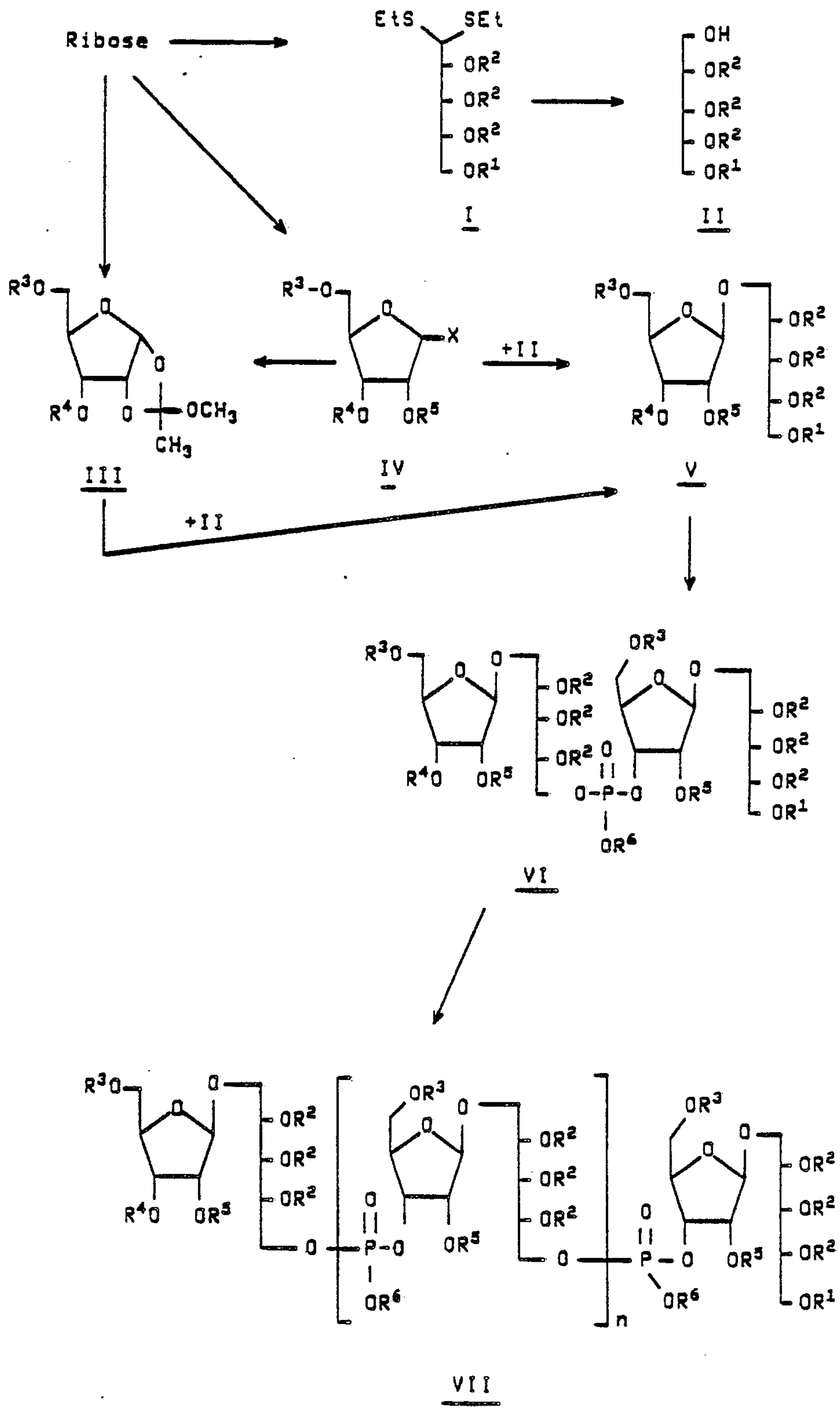

In accordance with the above reaction scheme, synthesis of a vaccine based on the structure of Haemophilus influenzae type b capsular polysaccharide can be described in the following seven steps:

(1) Construction of a ribitol of Formula II in which the $R^1$ group can be removed for coupling with an activated phosphorus while the $R^2$ groups are kept up to the point of final deblocking.

(2) Coupling of the ribitol of Formula II with a D-ribose derivative, special consideration being given to assure that only the $\beta$-anomer is formed.

(3) Protection of the three hydroxyl groups of the ribose unit such that the protecting group at 0-3 ($R^4$ of Formula V) may be removed in the presence of all other protecting groups.

(4) Introduction of a phosphate ester between 0-1 of one monomeric unit and 0-3′ of another unit, and assuring that the remaining oxygen of the phosphate is protected with functionality which can survive the duration of the synthesis ($R^6$ of Formula VI).

(5) Oligomerization to the structure of Formula VII.

(6) Introduction of an appropriate spacer unit at $R^4$ of Formula VII for attachment to an immunogenic carrier and removal of all protecting groups.

(7) Conjugation of the product to an immunogenic carrier.

Construction of the ribitol unit: The aldehyde function of D-ribose is first masked by the formation of a diethyldithioacetal (Formula I, $R^1$ and $R^2$ = H). The 5-hydroxyl group of I is selectively protected by using such reagents as t-butyldiphenylsilyl chloride, trityl chloride, or their derivatives (Formula I, $R^2$ = H, $R^1$ = trityl, 4-methoxytrityl, or t-butyldiphenylsilyl). In practice, the monomethoxytrityl group, introduced by treating diethyldithioribose (Formula I, $R^1$ and $R^2$ = H) with monomethoxytrityl chloride in the presence of silver nitrate or triethylamine in tetrahydrofuran, proved preferably for scale-ups. Subsequent benzylation using sodium hydride and benzyl bromide in dimethylformamide affords the fully protected D-ribose of Formula I ($R^1$ = monomethoxytrityl, $R^2$ = benzyl).

Once the 2, 3, and 4-hydroxy groups of Formula I have been protected, the monomethoxytrityl group can be removed, if desired, by mild acid hydrolysis and replaced with protecting groups additionally useful for construction of oligomeric units. Thus, esterification of I ($R^1$ = H, $R^2$ = benzyl) with trimethylacetyl chloride, etherification with bis(4-nitrophenyl)diazomethane, 4-methoxybenzyl chloride, or allyl bromide, or reaction with 4-methoxyphenol under Mitsunobu conditions, can generate the compounds listed in Table I. Conditions for removing these protecting groups selectively are also listed.

**Table I**

**5-O-Protected Ribose Derivatives**

**Formula I, $R^1$=Benzyl**

| $R^2$ | Cleavage Reagents |
|---|---|
| monomethoxytrityl | 3% trichloroacetic acid |
| trimethylacetyl | $CH_3NH_2$ |
| 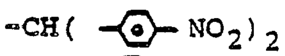 | $H_2/Pt$, pH=4 |
| 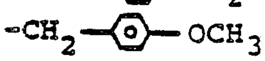 | 2,3-dichloro-5,6-dicyano-1,4-benzoquinone |
| allyl | 1) 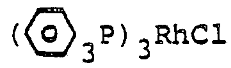 2) HgCl/HgO |
| 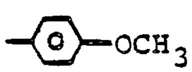 | ceric ammonium nitrate |

Hydrolysis of the dithioacetal function and reduction of the resulting aldehyde of the protected riboses can be achieved, using mercury (II) chloride and cadmium carbonate in acetone-water at room temperature. The resulting crude aldehydes are reduced using sodium cyanoborohydride in dimethoxyethane or sodium borohydride in methanol to yield the compounds of Formula II, $R^2$ = benzyl, $R^1$ = benzyl, monomethoxytrityl, trimethylacetyl, 4-methoxyphenyl, 4,4′-dinitrobenzhydryl, 4-methoxybenzyl and allyl.

Stereocontrolled glycosidation: Although numerous methods are available for stereospecific glycosidation of carbohydrates to yield exclusively the β-anomers, two such methods are particularly useful for the purposes of this invention. The first involves conversion of ribose to a diacetyl ortho ester [Formula III, $R^3$ and $R^4$ = acetyl, S. Hanessian, J. Banoub, Carbohydrate Res. 44, C14 (1975)], hydrolysis of the acetates with sodium methoxide (Formula III, $R^3$ and $R^4$ = H) followed by re-protection with t-butyldiphenylsilyl chloride (Formula III, $R^3$ and $R^4$ = t-butyldiphenylsilyl). This compound, when reacted with ribitols of Formula II in the presence of mercuric chloride as catalyst yields compounds of Formula V (eg. $R^1$ = monomethoxytrityl, $R^2$ = benzyl, $R^3$ and $R^4$ = t-butyldimethylsilyl, $R^5$ = acetyl) which offer the advantage of differential protection on the two secondary hydroxyl groups of the ribose residue.

A more preferred route for preparing the monomeric β-ribosylribitols of Formula V is to convert ribose into β-methyl 2,3-isopropylidine ribose using the method of N. J. Leonard and K. L. Carraway, J. Het. Chem. 3, 485 (1966), convert this intermediate to its 5-benzyl counterpart in the presence of benzyl bromide and base, then generate 5-benzyltriacetylribose in a two-step procedure wherein the methyl and benzylidine groups are removed under acidic conditions followed by treatment of the intermediate with acetic anhydride to yield a compound of Formula IV ($R^3$ = benzyl, $R^4$ and $R^5$ = acetyl, X = O-acetyl). This compound, when first treated with hydrogen bromide, then N,N-dimethylformamide dimethylacetal yields the compound of Formula III ($R^3$ = benzyl, $R^4$ = acetyl). Replacement of the 3-O-acetyl group by an allyl group (Formula III, $R^3$ = benzyl, $R^4$ = allyl) generates a key intermediate for stereospecific glycosylation. This is achieved by first treating the riboside with trimethylsilyl chloride followed by silver perchlorate in acetonitrile. Addition of the ribitol of Formula II ($R^2$ = benzyl, $R^1$ = allyl) generates the β-ribosylribitol of Formula V ($R^2$ and $R^3$ = benzyl, $R^1$ and $R^4$ = allyl, and $R^5$ = acetyl) in high yield and good anomeric purity. Similarly, an overwhelming predominance of a β-anomeric linkage can be generated employing a modification of the classical Koenigs-Knorr glycosidation reaction. When compounds of Formula IV ($R^3$, $R^4$, and $R^5$ = acetyl, X = Cl or Br) are reacted with compounds of Formula II ($R^2$ = benzyl, $R^1$ = benzyl or trimethylacetyl) in the presence of silver perchlorate and 4A molecular sieves in acetonitrile at 0° to 60° C, disaccharides of Formula V ($R^3$, $R^4$, and $R^5$ = acetyl, $R^2$ = benzyl, $R^1$ = benzyl or trimethylacetyl) are produced. The disaccharides are further characterized by base hydrolysis followed by hydrogenation to yield the deprotected disaccharide of Formula V ($R^1$, $R^2$, $R^3$, $R^4$, and $R^5$ = H) which exhibited physical characteristics consistant with the same disaccharide as isolated from degradation of natural PRP.

Ribofuranose protecting group modification: In order to construct oligomers from disaccharides of Formula V, the ability to selectively and independently remove protecting groups $R^1$ or $R^4$ in the presence of all other protecting groups is essential. The direct approach, wherein the reactivity of the primary hydroxyl of the ribofuranose of Formula V is used to introduce a protecting group $R^3$ followed by selective functionalization of $R^4$ or $R^5$ of Formula V is not as preferable as an indirect approach wherein several exchanges of protecting groups results in generation of the desired intermediates. Aminolysis of the compound of Formula V ($R^1$ = trimethylacetyl, $R^2$ = benzyl, $R^3$, $R^4$, and $R^5$ = acetyl) followed by treatment of the intermediate ($R^1$ and $R^2$ as defined, $R^3$, $R^4$, and $R^5$ = H) with 1,3-dichloro 1,1,3,3-tetraisopropyldisiloxane in pyridine or dimethylformamide/imidazole yields a compound of Formula V ($R^1$ = trimethylacetyl, $R^2$ = benzyl, $R^3$, $R^4$ together = tetraisopropyldisiloxane-1,3-diyl, and $R^5$ = H). Selective protection of the 2′-hydroxyl group can be achieved by reaction of the previous compound with, for example, benzyloxymethyl chloride in the presence of tetra-n-butylammonium iodide and diisopropylethylamine in tetrahydrofuran for 4-6 days at around room temperature, or by reaction with t-butyldimethylsilyl chloride ($R^5$ = benzyloxymethyl or t-butyldimethylsilyl, respectively). Removal or the 3′,5′-disilylether from the compound of Formula V ($R^1$ = trimethylacetyl, $R^2$ = benzyl, $R^3$, $R^4$ together = tetraisopropyldisiloxane-1,3-diyl, $R^5$ = benzyloxymethyl) with tetrabutylammonium fluoride, followed by reaction with a trityl chloride or hindered silyl chloride such as t-butyldimethylsilyl chloride in tetrahydrofuran in the presence of silver nitrate and 4-(dimethylamino)pyridine yields a compound of Formula V ($R^1$ = trimethylacetyl, $R^2$ = benzyl, $R^3$ = t-butyldimethylsilyl, $R^4$ = H, and $R^5$ = benzyloxymethyl) which has proper orientation of protecting groups for oligomerization. A further adjustment of protecting groups can be achieved by treating the previous compound with lithium triethylborane in tetrahydrofuran to remove the trimethylacetyl group ($R^1$) followed by reaction with 4-methoxytrityl chloride to yield a compound of Formula V ($R^1$ = monomethoxy trityl, $R^2$ = benzyl, $R^3$ = t-butyldimethylsilyl, $R^4$ = H, and $R^5$ = benzyloxymethyl).

The ether building block for construction of oligomers requires that the 5-hydroxy function of the ribitol unit be unprotected (Formula V, $R^1$ = H). One such intermediate, prepared from the compound of Formula V ($R^1$-trimethylacetyl; $R^2$ = benzyl, $R^3$ = t-monomethoxytrityl, $R^4$ = t-butyldimethylsilyl, and $R^5$ = benzyloxymethyl) with lithium aluminum chloride in tetrahydrofuran, results in removal of $R^1$ selectively. An alternative method for preparing this type of intermediate is to react the compound of Formula V ($R^1$ = monomethoxytrityl, $R^2$ = benzyl, $R^3$ = butyldimethylsilyl, $R^4$ = H, and $R^5$ = benzyloxymethyl) with levulinic acid in the presence of dicyclohexylcarbodiimide which yields a compound of Formula V ($R^1$ = monomethoxytrityl, $R^2$ = benzyl, $R^3$ = t-butyldimethylsilyl, $R^4$ = levulinyl, and $R^5$ = benzyloxymethyl) which upon treatment with dilute acid generates a compound without a protecting group at 0-5 (Formula V, R′-H). Yet another method for selective functionalization of the 0-5 and 0-3′ positions is available when the protecting groups at these two positions are initially the same, as for example in the compound of Formula V ($R^2$, $R^3$, and $R^5$ = benzyl, $R^1$ and $R^4$ = allyl). Both allyl groups are removed first by isomerizing the allyl bond to that of a vinyl ether using tris(triphenylphosphine) rhodium (II) chloride followed by cleavage in the presence of mercuric chloride and mercuric oxide to yield a compound of Formula V ($R^2$, $R^3$, and $R^5$ = benzyl, $R^1$ and $R^4$ = H). Treatment of the latter compound with monomethoxytrityl chloride in pyridine in

the presence of dimethylaminopyridine selectively protects the 0-5 hydroxyl group (Formula V, $R^1$ = monomethoxytrityl, $R^2$, $R^3$, and $R^5$ = benzyl, $R^1$ and $R^4$ = H). Further reaction of this compound with levulinic acid in the presence of 4-(N,N-dimethylamino)pyridine and dicyclohexylcarbodiimide followed by treatment with trichloroacetic acid yields a compound with a free hydroxyl group at 0-5 of the ribitol (Formula V $R^2$, $R^3$, and $R^5$ = benzyl, $R^4$ = levulinyl, $R^1$ = H).

Introduction of phosphate functionality: Numerous methods for introducing phosphate functionality between carbohydrate residues are available, particularly as applied to the synthesis of DNA and RNA fragments, so the method presented in this application should not be considered as limiting. In addition, although the first phosphorus-oxygen bond formation as described is to 0-3′ of the ribose unit, and the second phosphorus-oxygen bond formation is achieved to the 0-5 of ribitol, a reversal of this order should be considered as within the scope of this invention.

Thus, in accordance with the reaction scheme, a compound of Formula V ($R^1$ = trimethylacetyl, $R^2$ = benzyl, $R^3$ = monomethoxytrityl, $R^4$ = H, and $R^5$ = benzyloxymethyl) is reacted with diisopropylmethyl phosphoramidic chloride to yield the phosphoramidite of Formula V ($R^1$ = trimethylacetyl, $R^2$ = benzyl, $R^3$ = monomethoxytrityl, $R^4$ = diisopropylmethylphosphoramidyl, and $R^5$ = benzyloxymethyl). Synthesis of the fully-protected dimer is achieved by reacting the phosphoramidite with the alcohol of Formula V ($R^1$ = H, $R^2$ = benzyl, $R^3$ = monomethoxytrityl, $R^4$ = t-butyldimethylsilyl, and $R^5$ = benzyloxymethyl) in anhydrous acetonitrile in the presence of tetrazole, followed by oxidation with iodine, which generates the phosphotriester of Formula VI ($R^1$ = trimethylacetyl, $R^2$ = benzyl, $R^3$ = monomethoxytrityl, $R^4$ = t-butyldimethylsilyl, $R^5$ = benzyloxymethyl, and $R^6$ = methyl). Similarly, reaction of the alcohol of Formula V ($R^1$ = monomethoxytrityl, $R^2$ = benzyl, $R^3$ = t-butyldimethylsilyl, $R^4$ = H, and $R^5$ = benzyloxymethyl) with diisopropylmethylphosphoramidic chloride, followed by the reaction of the primary alcohol of the compound of Formula V ($R^1$ = H, $R^2$ = benzyl, $R^3$ = t-butyldimethylsilyl, $R^4$ = levulinyl, and $R^5$ = benzyloxymethyl) and oxidizing the intermediate phosphite with iodine generates the dimer of Formula VI ($R^1$ = monomethoxytrityl, $R^2$ = benzyl, $R^3$ = t-butyldimethylsilyl, $R^4$ = levulinyl, $R^5$ = benzyloxymethyl, and $R^6$ = methyl). Formation of the phosphorus-oxygen bonds is not restricted to the use of diisopropylmethylphosphoramidic chloride as coupling reagent. For example, the compound of Formula V ($R_1$ = monomethoxytrityl, $R^2$, $R^3$, and $R^5$ = benzyl, and $R^4$ = H), when reacted with 2-cyanoethyl-N,N-diisopropylphosphoramidic chloride likewise yields an intermediate useful for oligomerization (Formula V, $R^1$ = monomethoxytrityl, $R^2$, $R^3$, and $R^5$ = benzyl, $R^4$ = 2-cyanoethyldiisopropylphosphoramidyl). The intermediate, when reacted with a compound containing a free hydroxyl group at C-5 of ribitol (Formula V, $R^1$ = H, $R^2$, $R^3$, and $R^5$ = benzyl, $R^4$ = levulinyl) in acetonitrile in the presence of tetrazole, followed by oxidation with iodine as described before, leads to formation of the dimer of Formula VI ($R^1$ = monomethoxytrityl, $R^2$, $R^3$, and $R^5$ = benzyl, $R^4$ = levulinyl, and $R^6$ = 2-cyanoethyl). The monomethoxytrityl and levulinyl groups of the dimers of Formula VI can be removed independently and selectively by treating the compounds with dilute acid or hydrazine in a mixture of acetic acid and pyridine, respectively. Furthermore, the remaining protecting groups can be removed as follows: t-butylamine at elevated temperature removes the methyl group, and ammonia removes the 2-cyanoethyl protecting group ($R^6$), tetrabutylammonium fluoride removes the t-butyldimethylsilyl groups ($R^3$), and catalytic hydrogenation, with, for example, palladium-on-carbon as catalyst removes the benzyl and benzyloxymethyl groups to generate the fully-deprotected dimer of Formula VI ($R^1$, $R^2$, $R^3$, $R^4$, and $R^5$ = H, $R^6$ = counterion, e.g., $Na^+$).

Oligomerization of disaccharide units: Oligomerization can be achieved by any number of methods or combinations of strategies to prepare products of Formula VII, as long as the protecting groups $R^1$ and $R^4$ can be removed selectively and independently. Although preparation of a tetramer is described below, the same procedure can be applied to synthesis of a trimer from appropriate manipulations between compounds of Formulas V and VI, a hexamer can be prepared by appropriate modifications of two trimers, three dimers, six monomers, or any combinations thereof, an octomer can be prepared by combining two tetramers, four dimers, eight monomers, or any combinations thereof, and so forth. Hydrolysis of the levulinyl group from the compound of Formula VI ($R^1$ = monomethoxytrityl, $R^2$ = benzyl, $R^3$ = t-butyldimethylsilyl, $R^4$ = levulinyl, $R^5$ = benzyloxymethyl, and $R^6$ = methyl) under the conditions described previously and reaction of the product with diisopropylmethylphosphoramidic chloride generates the compound of Formula VI ($R^1$ = monomethoxytrityl, $R^2$ = benzyl, $R^3$ = t-butyldimethylsilyl, $R^4$ = diisopropylmethylphosphoramidyl, $R^5$ = benzyloxymethyl, and $R^6$ = methyl). This compound in turn is reacted with a second dimer of Formula VI ($R^1$ = H, $R^2$ = benzyl, $R^3$ = t-butyldimethylsilyl, $R^4$ = levulinyl, $R^5$ = benzyloxymethyl) in dry acetonitrile in the presence of tetrazole, followed by oxidation with iodine, to yield the fully-protected tetramer of Formula VII ($R^1$ = monomethoxytrityl, $R^2$ = benzyl, $R^3$ = t-butyldimethylsilyl, $R^4$ = levulinyl, $R^5$ = benzyloxymethyl, and $R^6$ = methyl, and n = 2). Again, $R^1$ or $R^4$ can be removed selectively and independently from the latter compound for additional oligomerization, or, if desired, all protecting groups can be removed to yield a

compound of Formula VII ($R^1$, $R^2$, $R^3$, $R^4$, and $R^5$ = H, $R^6$ = counterion, e.g., $Na^+$, and n = 2) using the conditions described previously for deprotection of the dimer.

Introduction of spacer unit: One of the many distinguishing features of this invention is that it not only teaches the preparation of oligomeric units of PRP, but also provides for introduction of a spacer unit for conjugation to immunogenic proteins. Moreover, the spacer unit can be derived from the structure of PRP itself and as such provides the opportunity to use a spacer which confers no undesired immunological effect itself. Although the construction of a ribitol-based fragment is described, other spacers of homologous or different structures are considered within the scope of this invention.

For synthesis of one such spacer, a suspension of D-ribose, benzaldehyde, benzyl alcohol, and catalytic amount of sulfuric acid is vigorously shaken to yield benzyl 2,3-benzylidineribofuranoside. This compound is reacted with diisopropylmethylphosphoramidic chloride to yield benzyl 2,3-benzylidine-5-(diisopropylmethyl)phosphoramidylribofuranose. The spacer is then reacted with the compound of Formula VII (($R^1$ = monomethoxytrityl, $R^2$ = benzyl, $R^3$ = t-butyldimethylsilyl, $R^4$ = H, $R^5$ = benzyloxymethyl, $R^6$ = methyl, and n = 2), followed by oxidation with iodine, to yield the compound of Formula VII ($R^1$ = monomethoxytrityl, $R^2$ = benzyl, $R^3$ = t-butyldimethylsilyl, $R^4$ = benzyl 2,3-benzylidineribofuranos-5-yl-methoxyphosphonyl, $R^5$ = benzyloxymethyl, $R^6$ = methyl, and n = 2) wherein the spacer has been incorporated at $R^4$ of Formula VII and contains a masked aldehyde for conjugation to immunogenic proteins.

Removal of protecting groups and conjugation to immunogenic carriers: Although removal of the protecting groups can be done in a different order, it is advantageous, for reasons of stability and yield, to adhere to the following sequence: removal of $R^6$ using t-butylamine at elevated temperature, removal of silyl groups, e.g., $R^3$, using tetrabutylammonium fluoride in a solvent such as tetrahydrofuran, and removal of benzyl and benzyloxymethyl groups by catalytic hydrogenation in a solvent system such as methanol-water. Introduction of the appropriate phosphate counterion (e.g., $R^6$ = $Na^+$) can be achieved using an ion-exchange resin. The above sequence generates a compound of Formula VII ($R^1$, $R^2$, $R^3$, $R^5$ = H, $R^4$ = ribos-5-ylphospho, $R^6$ = counterion, e.g., $Na^+$, and n = 2), suitable for conjugation to an immunogenic protein. This is accomplished most conveniently with reference to the last-mentioned compound, by reacting the compound with an amino-containing immunogenic macromolecule. In this case, a reaction occurs between the aldehyde of the spacer and the amine group(s) of the macromolecule to form imines, which can be reduced to aliphatic amine linkages, if necessary, by the addition of a reducing agent such as sodium cyanoborohydride. Other methods for conjugating the compounds to immunogenic macromolecules are likewise contemplated by this invention. Mild oxidation of the aldehyde of the spacer described above yields a carboxylic acid, which can be conjugated to an amine-containing macromolecule through the use of, for example, carbodiimides, or by first converting the carboxyl group to its corresponding hydroxysuccinimide ester. The hydroxysuccinimide ester can be further converted to, for example, an acyl hydrazine, which can be conjugated to immunological carriers which have been modified to contain aldehyde or ketone functionality. If, however, the spacer group (Formula VII, $R^4$) is introduced containing a terminal sulfhydryl functionality, conjugation to a sulfhydryl-containing immunogenic macromolecule can be achieved under oxidative conditions, or by first derivatizing the macromolecule with a Michael acceptor such as, for example, an m-maleimidobenzoyl group. If the spacer group is introduced such that it contains a primary amino group at its terminus, conjugation can be accomplished by activating the carboxyl groups of the immunological carrier toward neucleophilic displacement.

## Example 1

### Diethyldithio-D-ribose

A mixture of 275 g of ribose and 275 ml of concentrated hydrochloric acid was cooled in an ice-water bath, and to this was added 275 ml of ethane thiol. The reaction was stored in a refrigerator overnight, diluted with ethyl acetate and water, and neutralized by the cautious addition of an aqueous sodium carbonate solution. The organic layer was washed with brine, dried, and seeded, whereupon the desired compound crystallized, m.p. = 81-83 °C.

## Example 2

5-O-[(4-Methoxyphenyl)diphenylmethyl]-D-ribose diethyldithioacetal

To a stirred solution of 238 g of ribose diethyldithioacetal and 143 ml of triethylamine in 2 liters of tetrahydrofuran was added 316 g of 4-methoxytrityl chloride in portions over a period of about 45 minutes. After overnight stirring at room temperature, the reaction was filtered to remove precipitated triethylamine hydrochloride and evaporated to 612 g of an orange oil. The oil was re-dissolved into 1.5 liters of ether and washed with 400 ml each of 5% sodium bicarbonate, water, and brine. The organic layer was dried and evaporated to 514 g of an orange oil, which was used without further purification. For preparation of an anlytical sample, a portion of the product was chromatographed on silica gel, eluting first with 20% ethyl acetate in hexanes, then 30% ethyl acetate in hexanes, yielding the desired compound as a light yellow oil, $[\alpha]_D^{26} = 4^\circ \pm 1$ (c=1.19, chloroform).

Example 3

5-O-[(4-Methoxyphenyl)diphenylmethyl]-2,3,4-tris-O-(phenylmethyl)-D-ribose-diethyldithioacetal

A 266 g portion of the product from Example 2 was dissolved into one liter of dimethoxyethane and added dropwise to 121 g of stirred hexane-washed 60% sodium hydride suspended into 500 ml of dimethoxyethane. During addition, the flask was cooled in an ice-water bath and argon was bubbled into the reaction. After all of the starting material had been added and hydrogen evolution had ceased, 284 g of benzyl bromide was added dropwise over a period of about 1 hour. After stirring for about 18 hours at room temperature, residual sodium hydride was destroyed by the dropwise addition of 200 ml of ethanol. The reaction was filtered through a pad of celite, the pad was washed thoroughly with ether, and the solvents were evaporated. The crude product was dissolved into 1 liter of ether and washed with three portions of water and once with 500 ml of brine. The organic layer was dried and evaporated to give 397 g of crude product as an orange oil which was used without further purification. For preparation of an analytical sample, 5 g of the product was pre-adsorbed onto 8 g of silica gel and chromatographed on 350 g of silica gel, eluting with 5% ethyl acetate in hexanes. This yielded a faintly yellow oil, $[\alpha]_D^{26} = 11^\circ \pm 3$ (c=0.375, chloroform).

Example 4

2,3,4-Tris-O-(phenylmethyl-D-ribose-1,1-diethyldithioacetal

To 392 g of the crude product from Example 3 in 1 liter of ether/methanol (1:1) was added 15 g of toluenesulfonic acid monohydrate and the solution was left to stir at room temperature overnight. Sodium bicarbonate (10 g) was added in portions to the stirred solution, the precipitate was filtered off, and the solvents were evaporated to yield an orange oil. The crude product was purified in batches on silica gel, using a gradient of 5-15% ethyl acetate in hexanes to yield a light yellow oil, $[\alpha]_D^{26} = 35^\circ \pm 1$ (c=1.1, chloroform).

Example 5

2,3,4-Tris-O-(phenylmethyl)-5-O-(trimethylacetyl)-D-ribose diethyldithioacetal

To a solution of 800 mg of the product from Example 4, 361 mg of pyridine and 28 mg of 4-dimethylaminopyridine in 8 ml of methylene chloride, at 0° C, was added 275 mg of trimethylacetyl chloride. The resulting mixture was stirred at 0° C for 30 minutes and at room temperature for 2.5 hours. Aqueous 10% sodium bicarbonate was added. The mixture was concentrated at reduced pressure, the residue was diluted with methylene chloride and washed with dilute hydrogen chloride solution and brine. The organic phase was dried with anhydrous sodium sulfate. After removal of solvent under diminished pressure, the residue was purified on a short column of silica gel to give the desired ester; $^1$H NMR (300 MHz) 1.18 (t, 3H, $SCH_2CH_3$), 1.19 (t, 3H, $SCH_2CH_3$), 1.25 [s, 9H, $C(CH_3)3$], 2.51-2.69 (m, 4H, $SCH_2CH_3$), 3.93 (dd, 1H, $C_2$-H), 4.05 (m, 1H, $C_4$-H), 4.17 (d, 1H, $C_1$-H), 4.19-4.24 (m, 1H, $C_3$-H; 1H, $C_5$-H), 4,46 (dd, 1H, $C_5$-H), 4.63-5.12 (m, 6H, $OCH_2Ph$), 7.21-7.40 (m, 15H, Ar-H).

Example 6

5-O-Bis[(4-nitrophenyl)methyl]-2,3,4-tris-O-(phenylmethyl)-D-ribose diethyldiethioacetal

To a septum-sealed flask containing 65 mg of the alcohol of Example 4 and 106 mg of bis(4-nitrophenyl)diazomethane in 7 ml of dichloromethane, under argon at -40° C, was added 0.032 ml of freshly distilled borontrifluoride etherate, dropwise. The resulting red solution was stirred at -40° C for 18 hours. Aqueous sodium bicarbonate was added, followed by dichloromethane. The organic phase was washed again with aqueous sodium bicarbonate and water and dried over anhydrous sodium sulfate. The solvent was evaporated in vacuo. Flash chromatography of the residue on silica gel gave the desired product as a colorless oil. $^1$H NMR (200 MHz) 1.15 and 1.17 (2 t, 6H, $SCH_2CH_3$), 2.63 and 2.65 (2 q, 4H, $SCH_2CH_3$), 3.62 (d, 2H, $C_5$-H), 3.96 (dd, 1H, $C_2$-H), 4.15 (m, 2H, $C_{3,4}$-H), 4.21 (d, 1H, $C_1$-H), 4.54-4.95 (m, 6H, $CH_2Ph$), 5.87 [s, 1H, $CH(PhNO_2)_2$], 7.22-7.40 (m, 19H, Ar-H), 8.07 (m, 4H, $C_{3'},_{5'}$-H on $PhNO_2$).

Example 7

5-O-[(4-Methoxyphenyl)methyl]-2,3,4-tris-O-(phenylmethyl)-D-ribose diethyldithioacetal

To a solution of 350 mg of the alcohol of Example 4 in dimethylformamide at 0° C, was added 31 mg of sodium hydride. The solution was stirred under nitrogen for 30 minutes at room temperature and then cooled to 0° C. 4-Methoxybenzyl chloride (156 mg) in 2 ml of dimethylformamide was added dropwise. The resulting mixture was allowed to warm to ambient temperature and stirring continued for 4 hours. Ice-cold water was carefully added to the reaction mixture and the solution was concentrated at reduced pressure. The residue was diluted with water and extracted with diethyl ether. The combined extracts were dried, evaporated, and the residue was chromatographed on silica gel to yield the desired compound as a colorless oil. $^1$H NMR (200 MHz): 1.19 and 1.22 (2 t, 6H, $SCH_2CH_3$), 2.63 (q, 4H, $SCH_2CH_3$), 3.71 (m, 2H, $C_5$-H), 3.79 (s, 3H, $OCH_3$), 3.98 (dd, 1H, $J_{2,1}$ = 3.2 Hz, $C_2$-H), 4.11 (m, 4H, $C_{3,4}$-H), 4.24 (d, 1H, $J_{2,1}$ = 3.2 Hz, $C_1$-H), 4.40 (s, 2H, $CH_2OPh$), 4.57-4.95 (m, 6H, $CH_2OPh$), 6.84 (d, 2H, J = 8.2 Hz, $C_{3'},_{5'}$-H on $PhOCH_3$), 7.19-7.40 (m, 17H, Ar-H).

Example 8

5-O-[(4-Methoxy)phenyl]-2,3,4-tris-O-(phenylmethyl)-D-ribose diethyldithioacetal

To a solution of 210 mg of the alcohol of Example 4, 136 mg of triphenylphosphine and 149 mg of 4-methoxyphenol in 1.5 ml of tetrahydrofuran was added 90.5 mg of diethyl azodicarboxylate in 1 ml of tetrahydrofuran, dropwise. The resulting mixture was refluxed for 3 hours. The solvent was evaporated and the residue was chromatographed to yield the desired dithioacetal as an oil. High resolution mass spec: (CI, $NH_3$, m/z) for $C_{33}H_{37}O_6$ $(M + H^+)$, calcd 529.2590, found 529.2590.

## Example 9

### 2,3,4-Tris-O-(phenylmethyl)-5-O-(prop-2-enyl)-D-ribose-1,1-diethyldithioacetal

A 2.51 g sample of sodium hydride as a 60% oil dispersion was added to a solution of 22 g of the alcohol from Example 4 and 1.545 g tetra-n-butylammonium iodide in 200 ml of tetrahydrofuran. After the mixture was stirred at room temperature for 30 minutes, 16 ml of allyl bromide was added and stirring was continued for an additional 16 hours. Water was carefully added and the solvents were evaporated. The resultant oil was dissolved in ether, and this was washed with brine. The ether layer was dried and evaporated to yield the crude desired product.

## Example 10

### 5-O-[(4-Methoxyphenyl)diphenylmethyl]-2,3,4-tris-O-(phenylmethyl)-D-ribitol

A mixture of 3.112 g of the dithioacetal of Example 3, 2.118 g of mercury (II) chloride and 2.112 g of yellow mercury oxide in 55 ml of 6% aqueous acetone was stirred at room temperature for 2 hours. Hexanes were added and the mixture was filtered through Celite. The filtrate was concentrated and again filtered through Celite. The organic phase was washed with 5% aqueous potassium iodide and water. After drying, the solvent was evaporated to give the crude aldehyde as an oil. To this aldehyde in 50 ml of methanol were added 4.426 g of sodium borohydride and 232 mg of sodium methoxide. After 2 hours, the reaction mixture was diluted with chloroform and filtered through Celite. The combined filtrates were washed with water, dried, and evaporated to afford the crude product which was purified by flash chromatography to give the desired alcohol $[\alpha]_D^{20} = -18.4^\circ$ (c = 10.9, chloroform).

## Example 11

### 2,3,4-Tris-O-(phenylmethyl)-5-O-(trimethylacetyl)-D-ribitol

A mixture of 860 mg of the dithioacetal of Example 5, 956 mg of mercury(II) chloride and 608 mg of cadmium carbonate in 30 ml of 5:1 (v/v) acetone-water, was refluxed for 2 hours. The solvent was evaporated, the residue was diluted with methylene chloride and filtered through Celite. The combined filtrate and washings were washed with aqueous potassium iodide and brine and dried. Removal of solvent at reduced pressure afforded the desired aldehyde. The aldehyde (680 mg) was dissolved in 50 ml of 1,2-dimethoxyethane and 170 mg of sodium cyanoborohydride was added. The resulting mixture was stirred at room temperature for 5-6 hours. The solution was filtered through Celite and evaporated. Chromatography of the residue on silica gel gave the desired alcohol. $[\alpha]_D^{22} = +4.39^\circ$ (c = 11.75, chloroform).

Example 12

5-O-[(4-Methoxy)phenyl]-2,3,4-tris-O-(phenylmethyl)-D-ribitol

The dithioacetal of Example 8 hydrolyzed and reduced as described in Example 10 yielded the title compound as an oil. Mass spectrum: (CI, $NH_3$, %) 546 (M + $NH_4$ +, 100), 529 (M + $H^+$, 62.2).

Example 13

5-O-Bis[(4-nitrophenyl)methyl]-2,3,4-tris-O-(phenylmethyl)-D-ribitol

The dithioacetal of Example 6 was treated by the method of Example 11 to yield the title alcohol as a pale yellow oil. Mass spectrum: (CI, $NH_3$, %) 679 (M + $H^+$, 12.2), 422 [$MH^+$ - $CH(PhNO_2)_2$, 100].

Example 14

5-O-[(4-Methoxyphenyl)methyl]-2,3,4-tris-O-(phenylmethyl)-D-ribitol

The dithioacetal of Example 7 was treated by the method of Example 11, yielding the title compound as an oil, $^1$H NMR (200 MHz) 2.29 (br s, 1H, OH), 3.64-3.73 (br m, 5H), $C_{1,2,5}$-H), 3.78 (s, 3H, $OCH_3$), 3.86 (br q, 1H, $C_4$-H), 3.95 (br q, 1H, $C_3$-H), 4.42 (s, 2H, $OCH_2Ph$), 4.56 (s, 2H, $OCH_2Ph$), 4.63-4.68 (m, 4H, $OCH_2Ph$), 6.84 (d, 2H, J=8.6 Hz, $C_3{'}$,$5'$-H on $PhOCH_3$), 7.20-7.33 (m, 17H, Ar-H).

Example 15

2,3,4-Tris-O-(phenylmethyl)-5-O-(prop-2-enyl)-D-ribitol

A solution of 20 g of the crude product from Example 9 in 50 ml of acetonitrile was added to a stirred solution of 11.8 g of N-chlorosuccinimide and 15 g of silver nitrate in 300 mg of 70% aqueous acetonitrile at -15 °C. After 10 minutes, 100 ml of saturated ammonium acetate was added. The solution was filtered and evaporated to give an oil. This was dissolved into ether, washed with brine, dried, and again evaporated. The residue was dissolved into 200 ml of methanol, and 2.67 g of sodium borohydride was slowly added. After 30 minutes, the reaction was quenched by addition of acetone, then worked up as described in Example 11 to yield the desired compound. High resolution mass spectrum (CI, $NH_3$) for $C_{29}H_{35}O_5$ (M + $H^+$) calcd: 463.24836; found: 463.24845.

Example 16

1,2-O-Methoxyethylidene-α-D-ribose

A solution of 1.54 g of the orthoester 1,2-O-methoxyethylidene-3,5-di-O-acetyl-α-D-ribose [prepared according to the method described by S. Hanessian, J. Banoub, Carbohydrate Res. 44, C14(1975)] in methanol was treated with 10.6 ml of 0.05M sodium methoxide in methanol at room temperature for 1 hour. The reaction was neutralized, water was then added, and the mixture was filtered. The filtrate was co-evaporated with benzene three times to give an oily residue, which was used as such without further purification.

## Example 17

### 1,2-O-(Methoxyethylidene)-3,5-di-O-[(1,1-dimethylethyl) dimethylsilyl]-α-D-ribose

The silylation of the diol of Example 16 with t-butyldimethylsilyl chloride was done using the method described by E. J. Corey, A. Venkateswarlu, J. Am. Chem. Soc. 94, 6190 (1972). The crude product was purified by chromatography. The desired compound was obtained as white crystals, m.p. = 37-38° C.

## Example 18

### 2′-O-Acetyl-3′,5′-di-O-[(1,1-dimethylethyl)dimethylsilyl]-1′-β-D-ribofuranosyl-5-O-[(4-methoxyphenyl)-diphenylmethyl]-2,3,4-tris-O-(phenylmethyl)-D-ribitol

The orthoester of Example 17 (481 mg) and 720 mg of the ribitol of Example 10 were dissolved in 6 ml of nitromethane. Methanol was removed by continuous distillation at constant volume with continuous addition of nitromethane for 2 hours. Mercury (II) bromide (56.5 mg) was added, and the solvent was distilled off at constant volume with continuous addition of nitromethane for 3 hours. The mixture was filtered and concentrated, and the product was isolated by chromatography to give the desired glycoside as oil. Mass spectrum: (FAB, glycerol, %) 403 [$M^+$ - 693 (ribitol chain), 29.7].

## Example 19

### Methyl-2,3-di-O-(methylethylidene)-5-O-(phenylmethyl)-β-D-ribofuranoside

To a solution of 50 g of methyl-2,3-di-O-(methylethylidene)-β-D-ribofuranoside [prepared according to the method of N. J. Leonard, K. L. Carraway, J. Heterocyclic Chem. 3, 485 (1966)] and tetra-n-butylammonium iodide in 500 ml of tetrahydrofuran was added 10.2 g of sodium hydride as a 60% oil dispersion. After 30 minutes, 22 ml of benzyl bromide was slowly added. After 18 hours, 20 g of Florisil was added and the tetrahydrofuran was evaporated. The residue was washed thoroughly with pentane, and the oil resulting from evaporation of the pentane was distilled, bp = 130-135° C (0.1 mm) to provide the desired compound as a colorless oil.

## Example 20

### 1,2,3-Tri-O-acetyl-5-O-(phenylmethyl)-D-ribofuranoside

A solution of 20 g of the product from Example 19 in 300 ml of 70% acetic acid was heated at 80° C for 6 hours. The solvents were evaporated to yield an oil. This was dissolved into 300 ml of methylene chloride containing 44 ml of pyridine and 0.830 g of 4-(N,N-dimethylamino)pyridine, and 25 ml of acetic acid was slowly added. After stirring for 30 minutes, 200 ml of saturated aqueous sodium bicarbonate solution was added, followed by solid potassium carbonate until the solution was basic. The solution was washed with 1N hydrochloric acid to acidity, dried, and evaporated to a pale yellow oil. NMR shows this product to exist in an α to β ratio of 25 to 75.

## Example 21

### 3-O-Acetyl-1,2-O-(methoxyethylidine)-5-O-(phenylmethyl)-α-D-ribofuranoside

Dry hydrogen bromide gas was bubbled into a solution of 13.5 g of the product from Example 20 in 200 ml of methylene chloride at room temperature for 3 minutes. The solvent was evaporated, and the residue was taken up three times into 150 ml of toluene, evaporating each time. The resulting oil was dissolved into 200 ml of methylene chloride and 52 ml of dimethylformamide dimethyl acetal was added. After stirring for 3 hours, the solution was filtered through a pad of silica gel, washing with ethyl acetate. Evaporation yielded the desired compound as a brown oil. Mass spectrum (CI, $NH_3$, %): 356 ($M + NH_4^+$, 36.7); 307 ($M^+$-MeO, 100).

## Example 22

### 1,2-O-(Methoxyethylidine)-5-O-(phenylmethyl)-3-O-(prop-2-enyl)-α-D-ribofuranoside

To a solution of 11 g of the product from Example 21 in 200 ml of methanol at room temperature was aded 192 mg of sodium methoxide. After 3 hours, the solvent was evaporated, the residue was dissolved into 250 ml of benzene, and 2.84 g of sodium hydride was added as a 60% oil dispersion, followed by 24 ml of allyl bromide. After 18 hours, brine was slowly added to the mixture and this extracted with ether. The combined organic extracts were washed with brine, dried, and evaporated. Chromatography of the residue on silica gel gave the desired compound as a pale yellow oil. High resolution mass spectrum (CI, $NH_3$) for $C_{18}H_{25}O_6$ ($M + H^+$): calculated 337.16524; found 337.16511.

## Example 23

### 2,3,5-Tris-O-acetyl-β-D-ribofuranosyl bromide and chloride

Anhydrous hydrogen bromide was bubbled rapidly through a solution of tetra-O-acetyl-β-D-ribofuranose in dry methylene chloride at room temperature for 3 minutes. The solution was left at room temperature for an additional 3 minutes and rapidly evaporated to a syrup. The syrup was co-evaporated with dry benzene and methylene chloride three times. The pale yellow syrup bromide in the β-anomeric form exclusively, was used immediately for the next step.

The title chloride was prepared in a similar manner, except that after saturation with hydrogen chloride, the reaction solution was kept in a refrigerator overnight.

## Example 24

2′-O-Acetyl-5′-O-(phenylmethyl)-3′-O-(prop-2-enyl)-1′-β-D-ribofuranosyl-2,3,4-tris-O-(phenylmethyl)-5-O-(prop-2-enyl)-D-ribitol

Freshly distilled trimethylsilyl chloride (7.65 ml) was added to a solution of 6.75 g of the product from Example 22 in dry methylene chloride. After 1 hour, the solvent was evaporated, the resulting oil was dissolved into 10 ml of dry acetonitrile, and this was added to a suspension of 4.574 g of silver perchlorate and 9 g of powdered 4A molecular sieves in 20 ml of dry acetonitrile at -40° C. After the reaction had been stirred for 1 hour, 4.641 g of the product from Example 15 in 10 ml of acetonitrile was slowly added. After 4 hours, the reaction was filtered, evaporated, and the desired product was purified by chromatography on silica gel. $^1$H NMR (200 MHz): 2.14 (s, $-COCH_3$), 3.56-3.678 (m, 5H), 3.71-4.21 (m, 10H), 4.50-4.73 (m, 8H, $CH_2Ph$), 5.14-5.33 (m, 5H, $-CH=CH_2$), 5.64-5.94 (m, 2H, $-CH=$), 7.25-7.38 (m, 20H, -Ph).

Example 25

2′,3′,5′-Tris-O-acetyl-1′-β-D-ribofuranosyl-5-O-(trimethylacetyl)-2,3,4-tris-O-(phenylmethyl)-D-ribitol

A mixture of 3.185 g of dry silver perchlorate and 3.7 g of 4A molecular sieves in 35 ml of acetonitrile was stirred at room temperature for about 10 minutes, then cooled to -40° C. A solution of 4.304 g of the ribofuranosyl chloride of Example 23 in 25 ml of acetonitrile was added dropwise. After 40 minutes, a solution of 3.7 g of the ribitol of Example 11 in 30 ml of acetonitrile was added dropwise and the resulting yellow solution was stirred for 2 hours at -40° C. Sodium bicarbonate powder (3.0 g) was added and the mixture was allowed to warm to room temperature. The reaction was filtered through Celite, washed with methylene chloride and the combined filtrate and washings were evaporated to a syrup. Flash chromatography of the residue gave the desired product as an oil, $[\alpha]_D^{22}$ = -12.79° (c, 16.5, chloroform).

Example 26

2′,3′,5′-Tris-O-acetyl-1′-β-D-ribofuranosyl-2,3,4,5-tetrakis-O-(phenylmethyl)-D-ribitol

The title compound, prepared by the method of Example 25 from 2,3,5-tris-O-acetyl-β-D-ribofuranosyl chloride (Example 23) and 2,3,4,5-tetra-O-benzyl ribitol [prepared according to the method of Garegg, et al., Acta Chem. Scand. 27, 1595 (1973)] was isolated as an oil. $^1$H NMR (300 MHz) 1.98, 2.06 and 2.09 (3 s, 9H, 3 $COCH_3$), 3.68 (m, 4H), 3.86 (m, 4H), 4.09 (m, 1H), 4.26 (m, 2H), 4.49-4.77 (m, 8H, $CH_2Ph$), 4.94 (s, 1H, $C_1$′-H), 5.27 (d, 1H, $C_2$-H), 5.32 (t, 1H), $C_3$′-H), 7.30 (m, 20H, Ar-H).

Example 27

3′-O-(Prop-2-enyl)-2′,5′-di-O-(phenylmethyl)-1′-O-β-D-ribofuranosyl-5-O-(prop-2-enyl)-2,3,4-tris-O-(phenylmethyl)-D-ribitol

To a 3 g sample of the product from Example 24 dissolved in 25 ml of methanol was added 21 mg of sodium methoxide. After 2 hours, the methanol was evaporated, and 0.251 g of sodium hydride as a 60%

dispersion and 0.723 g of tetra-n-butylammonium iodide was added to the residue dissolved into 50 ml tetrahydrofuran. After 1 hour, 0.64 ml of benzyl bromide was added and stirring was continued overnight. After a workup as described in Example 9, the desired product was isolated. $^1H$ NMR (200 MHz): 3.54-3.70 (m, 5H), 3.95-3.98 (m, 10H), 4.28-4.32 (m, 1H, H-4), 4.491-4.68 (m, 10H, $CH_2Ph$), 5.04 (s, 1H, H-1), 5.13-5.31 (m, 4H, $=CH_2$), 5,81-5.97 (m, 2H -CH=), 7.24-7.37 (m, 25H, Ph).

## Example 28

### 2′,5′-Bis-O-(phenylmethyl)-1′-O-β-D-ribofuranosyl-2,3,4-tris-O-(phenylmethyl)-D-ribitol

A solution of 5.921 g of the product from Example 27, 0.673 g of tris(triphenylphosphine) rhodium (I) chloride, and 0.244 g of diazabicyclooctane in 75 ml of 10% aqueous ethanol and 25 ml of toluene was refluxed for 6 hours. The solvents were evaporated and replaced with 10% aqueous acetone, then 5.925 g of mercuric chloride and 4.727 g of mercuric oxide were added. After stirring at room temperature for 1 hour, the solution was filtered and the solvents were evaporated to produce an oil. The residue was dissolved into methylene chloride, washed with 5% aqueous potassium iodide, brine, dried, and again evaporated. Silica gel chromatography yielded the desired compound as an oil. $^1H$ NMR (200 MHz): 2.29 (br t, 1H, C-5′OH), 2.60 (d, 1H, C-3-OH, J=8.5Hz), 3.50-4.17 (m, 12H), 4.48-4.69 (m, 10H, $CH_2Ph$), 5.01 (s, 1H, H-1), 7.28-7.32 (m, 25H, Ph).

## Example 29

### 2′,5′-Bis-O-(phenylmethyl)-1′-O-β-D-ribofuranosyl-5-O-[(4-methoxyphenyl)diphenylmethyl]-2,3,4-tris-O-(phenylmethyl)-D-ribitol

To a solution of 3.609 g of the product from Example 28 in 80 ml of dry methylene chloride containing 1.20 ml of pyridine and 0.302 g of 4-(N,N-dimethylamino)pyridine at room temperature was added 1.985 g of monomethoxytrityl chloride. After 15 hours, the solution was washed with saturated aqueous sodium bicarbonate and brine, dried, and the solvents were evaporated. Chromatography of the residue on silica gel provided the desired product as a white foam. $^1H$ NMR (200 MHz): 2.57 (d, 1H, C-3 OH, J=8.7 Hz); 3.41-4.10 (m, 12H); 3.75 (s, 3H, OMe); 4.47-4.82 (m, 10H, $CH_2Ph$); 4.98 (s, 1H, H-1); 6.75 (d, 2H, H-3 & H-5 on Ph-$OCH_3$); 7.21-7.493 (m, 37H, Ph).

## Example 30

### 3′-O-(1,4-Dioxopentyl)-2′,5′-Bis-O-(phenylmethyl)-1′-O-β-D-ribofuranosyl-5-O-[(4-methoxyphenyl)-diphenylmethyl]-2,3,4-tris-O-(phenylmethyl)-D-ribitol

To a solution of 535 mg of the product from Example 29 in 25 ml of tetrahydrofuran containing 98 mg of 4-(N,N-dimethylaminopyridine) was added 0.08 ml of levulinic acid followed by 220 mg of dicyclohexylcarbodiimide dissolved in 2 ml of tetrahydrofuran. After the solution was stirred for 24 hours, the reaction was filtered and the solvents were evaporated. Chromatography of the residue on silica gel produced the desired compound as a white foam. $^1H$ NMR (200 MHz): 2.13 (s, 3H, $CH_3CO$-); 2.58-2.66 (m, 4H, -$COCH_2CH_2$-); 3.47 (m, 2H); 3.48 (d, 2H, H-5′, J=4.9Hz); 3.50 (m, 1H); 3.75 (s, 3H, $OCH_3$); 3.87 (m, 4H); 4.07 (dd, 1H, H-2, $J_{1,2}$=1.8Hz, $J_{2,3}$=4.9Hz); 4.31 (dd, 1H, H-4, $J_{3,4}$=10.9Hz, $J_{4,5}$=5.4Hz); 4.44-4.68 (m, 10H, $CH_2Ph$); 4.98 (d, 1H, H-1, J=1.9Hz); 5.13 (dd, 1H, H-3, $J_{2,3}$=5.27Hz, $J_{3,4}$=10.6Hz); 6.74 (d, 2H, m-Ar-H on

$PhOCH_3$); 7.20-7.48 (m, 37H, Ph).

Example 31

3′-O-(1,4-Dioxopentyl)-2′,5′-bis-O-(phenylmethyl)-1′-O-β-D-ribofuranosyl-2,3,4-tris-O-(phenylmethyl)-D-ribitol

A solution of 482 mg of the product from Example 30 in 10 ml of 3% trichloroacetic acid in methylene chloride was stirred for 15 minutes at room temperature. The resulting orange solution was quenched with 10% aqueous sodium bicarbonate. The mixture was diluted with methylene chloride, washed with brine, dried, and evaporated. Chromatography of the residue on silica gel yielded the desired product as a white foam. $^1$H NMR (200 MHz): 2.14 (s, 3H, $CH_3CO$-); 2.27 (br t, 1H, -OH); 2.62-2.68 (m, 4H, -$CH_2CH_2CO$-); 3.53 (d, 2H, H-5,5′, J = 5.0Hz); 3.73-4.07 (m, 7H, ribitol); 4.10 (dd, 1H, H-2, $J_{1,2}$ = 2.2Hz, $J_{2,3}$ = 5.1Hz); 4.33 (dd, 1H, H-4, $J_{3,4}$ = 10.5Hz, $J_{4,5}$ = 5.1Hz); 4.44-4.68 (m, 10H, $CH_2Ph$); 5.02 (d, 1H, H-1, J = 2.1Hz); 5.17 (dd, 1H, H-3, $J_{2,3}$ = 5.3Hz, $J_{3,4}$ = 10.6Hz); 7.27-7.30 (m, 25H, Ph).

Example 32

1′-O-β-D-Ribofuranosyl-5-O-(trimethylacetyl)-2,3,4-tris-O-(phenylmethyl)-D-ribitol

A solution of 1.27 g of the triacetate of Example 25 in 100 ml of ammonia-saturated methanol was stirred at 0° C for 1 day. The solvent was evaporated and the residue was diluted with ethyl acetate. The solution was treated with ion-exchange resin and filtered. Evaporation of the solvent and chromatography on silica gel gave the desired triol. $^1$H NMR (200 MHz) 1.11 [s, 9H, $C(CH_3)_3$], 3.15 (br s, 3H, 3 OH), 3.56-3.64 (m, 2H), 3.73-4.20 (br m, 9H), 4.33 (br t, 1H), 4.61-4.65 (m, 6H, $CH_2OPh$), 4.85 (s, 1H, $C_1$′-H), 7.24-7.35 (m, 15H, Ar-H).

Example 33

3′5′-O-Tetrakis-[(1-methylethyl)disiloxane]-1,3-diyl-1′-O-β-D-ribofuranosyl-5-O-(trimethylacetyl)-2,3,4-tris-O-(phenylmethyl)-D-ribitol

To a solution of 2.22 g of the triol of Examle 32 in 20 ml of dry pyridine at 0° C was added 1.21 ml of 1,3-dichloro-1,1,3,3-tetraisopropyldisiloxane, slowly. The resulting mixture was stirred at 0° C for 3 hours and then at room temperature for 1 hour. Water was added and the solution was concentrated at reduced pressure. Methylene chloride was then added, and the solution was washed with water, 5% $CuSO_4$ and brine and dried with anhydrous sodium sulfate. After evaporation, the product was purified using silica gel chromatography to give the desired alcohol as a crystalline solid. $^1$H NMR (200 MHz): 1.00-1.10 [m, 21H, $CH(CH_3)_2$], 1.18 [s, 9H, $C(CH_3)_3$], 2.97 (d, 1H, OH), 3.61-4.19 (br m, 12H), 4,52-4.75 (m, 6H, $CH_2OPh$), 4.88 (s, 1H, $C_1$′-H), 7.30 (m, 15H, Ar-H).

Example 34

3′5′-O-Tetrakis-[(1-methylethyl)disiloxane]-1,3-diyl-2′-O-[(phenylmethoxy)methyl]-1′-O-β-D-ribofuranosyl-5-O-(trimethylacetyl)-2,3,4-tris-O-(phenylmethyl)-D-ribitol

To a solution of 1.068 g of the alcohol from Example 33, 1.163 ml of diisopropylethylamine and 448 mg of tetra-n-butylammonium iodide in 20 ml tetrahydrofuran under argon at room temperature was added 0.844 mg of benzyl chloromethyl ether followed by 74 mg of 4-(N,N-dimethylamino)pyridine. The resulting yellow suspension was stirred 4 days, then 10% aqueous sodium bicarbonate sodium was added and the mixture was evaporated. The residue was dissolved in methylene chloride and washed with 10% aqueous sodium bicarbonate solution and water. After drying, the solvent was evaporated and the residue was purified by chromatography. $^1H$ NMR (200 MHz) 1.02 [br d, 21H, $CH(CH_3)_2$], 1.20 [s, 9H, $C(CH_3)_3$], 3.66 (m, 1H), 3.77-3.92 (br m, 4H), 3.97-4.08 (m, 2H), 4.11-4.22 (m, 2H), 4.50 (dd, 1H), 4.51 (m, 1H), 4,52-4.75 (m, 6H, $CH_2OPh$), 4,83 (s, 1H, $C_1'$-H), 4.34 and 4.44 (2 d, 2H, $OCH_2OBn$), 7.23-7.37 (m, 20H, Ar-H).

## Example 35

3'5'-O-Tetrakis-[(1-methylethyl)disiloxane]-1,3-diyl-2'-O-[(1,1-dimethylethyl)dimethylsilyl]-1'-O-β-D-ribofuranosyl-5-O-(trimethylacetyl)-2,3,4-tris-O-(phenylmethyl)-D-ribitol

The product of Example 33 (150 mg) was treated with 60 mg of t-butyldimethylsilyl chloride and 53 mg of imidazole in 3 ml of dimethylformamide. After 1 hour, the reaction was worked up in the usual manner to yield the desired silyl ether. $^1H$ NMR (300 MHz): 0.05 and 0.07 (2 s, 6H, 2 $SiCH_3$), 0.88 [s, 9H, $SiC(CH_3)_3$], 1.01 [m, 28H, 4 $SiCH(CH_3)_2$], 1.17 [s, 9H, $OC(CH_3)_3$], 3.70 (br d, 2H), 3.83-4.07 (br m, 7H), 4.16 (dd, 1H), 4.40 (dd, 1H), 4.52 (dd, 1H), 4.59-4.73 (m, 6H, $CH_2Ph$), 4.77 (s, 1H, $C_1'$-H), 7.28 (m, 15H, Ar-H).

## Example 36

2'-O-[(Phenylmethoxy)methyl]-1'-O-β-D-ribofuranosyl-5-O-(trimethylacetyl)-2,3,4-tris-O-(phenylmethyl)-D-ribitol

To a solution of 1.18 g of the product from Example 34 in 20 ml of tetrahydrofuran was added 842 mg of tetra-n-butylammonium fluoride trihydrate. The reaction mixture was stirred for 1 hour and water was added. The mixture was concentrated, and the residue was extracted with chloroform. The combined extracts were washed with brine and dried with anhydrous sodium sulfate. After evaporation of the solvent, chromatography of the residue gave the desired diol as an oil. $^1H$ NMR (200 MHz) 1.14 [s, 9H, $C(CH_3)_3$], 2.37 (dd, 1H, 5'-OH), 2.60 (d, 1H, 3'-OH), 3.50-4.15 (br m, 11H), 4.36 (m, 1H), 4.41-4.70 (m, 8H, $CH_2OPh$), 4.84 (s, 2H, $OCH_2OBn$), 4.95 (s, 1H, $C_1'$-H), 7.19-7.34 (m, 20H, Ar-H).

## Example 37

5'-O-[(4-Methoxyphenyl)diphenylmethyl]-2'-O-[(phenylmethoxy)methyl]-1'-O-β-D-ribofuranosyl-5-O-(trimethylacetyl)-2,3,4-tris-O-(phenylmethyl)-D-ribitol

To a solution of 765 mg of the product from Example 36 in 30 ml of tetrahydrofuran was added 223 mg of silver nitrate, 399 mg of pyridine and 405 mg of monomethoxy trityl chloride, successively. The resulting mixture was stirred at room temperature for 4 hours and filtered through Celite, washing with methylene chloride. The filtrate was concentrated and 10% sodium bicarbonate was added. This was extracted with methylene chloride and dried. After evaporation and purification by chromatography, the desired product

was obtained as an oil. Mass spectrum: (FAB, DEA, m/e, %) 526 ($MH^+$ - OR, R=ribitol chain, 0.5), 631 (526 + DEA, 0.14).

Example 38

3′-O-[(1,1-Dimethylethyl)dimethylsilyl]-5′-O-[(4-methoxyphenyl)diphenylmethyl]-2′-O-[(phenylmethoxy)methyl]-β-D-ribofuranosyl-2,3,4-tris-O-(phenylmethyl)-5-O-(trimethylacetyl)-D-ribitol

The product of Example 37 was reacted with t-butyl-dimethylsilyl chloride as described in Example 35 to yield the desired compound as an oil. $^1H$ NMR (300 MHz) 0.1 and 0.03 (2 s, 6H, 2 $SiCH_3$), 0.77 [s, 9H, $SiC(CH_3)_3$], 1.19 [s, 9H, $OC(CH_3)_3$], 3.13 (dd, 1H), 3.32 (dd, 1H), 3.71 (s, 3H, $OCH_3$), 3.87 (m, 4H), 3.99 (d, 1H, $C_2$-H), 4.03 (m, 1H), 4.18 (m, 2H), 4.29 (dd, 1H, $C_3'$-H), 4.50 (m, 1H), 4.45-4.71 (m, 8H, $CH_2Ph$), 4.84 and 4.89 (2 d, 2H, $OCH_2OBn$), 5.11 (s, 1H, $C_1'$-H), 6.77 (d, 2H, $C_{3,5}$-H on $PhOCH_3$), 7.13-7.50 (br m, 32H, Ar-H).

Example 39

3′-O-[(1,1-Dimethylethyl)dimethylsilyl]-5′-O-[(4-methoxyphenyl)diphenylmethyl]-2′-O-[(phenylmethoxy)methyl]-β-D-ribofuranosyl-2,3,4-tris-O-(phenylmethyl)-D-ribitol

To a solution of the product from Example 38 (250 mg) in 2 ml of tetrahydrofuran at room temperature was added 9.1 mg of lithium aluminum hydride. After 30 minutes, 10% sodium bicarbonate solution was added, and the mixture was filtered through Celite. After evaporation, the residue was chromatographed on silica gel to give the desired alcohol as an oil. $^1H$ NMR (300 MHz) -0.03 and -0.16 (2 s, 6H, 2 $SiCH_3$), 0.81, [s, 9H, $SiC(CH_3)_3$], 2.25 (t, 1H, OH), 3.13 (dd, 1H), 3.34 (dd, 1H), 3.71 (s, 3H, $OCH_3$), 3.72 (m, 3H), 3.87 (m, 3H), 4.00 (d, 1H, $C_2'$-H), 4.03 (m, 1H), 4.21 (m, 1H), 4.31 (dd, 1H, $C_3$-H), 4.42-4.72 (br m, 8H, $OCH_2Ph$), 4.82 and 4.87 (2 d, 2H, $OCH_2OBn$), 5.13 (s, 1H, $C_1'$-H), 6.97 (d, 2H, $C_{3,5}$-H on $PhOCH_3$), 7.13-7.50 (m, 32H, Ar-H).

Example 40

5′-O-[(1,1-Dimethylethyl)dimethylsilyl]-2′-O-[(phenylmethoxy)methyl]-β-D-ribofuranosyl-2,3,4-tris-O-(phenylmethyl)-5-O-(trimethylacetyl)-D-ribitol

The mixture of the diol from Example 36 (9.860 g), 3.646 g of silver nitrate and 1.589 g of dimethylaminopyridine in 150 ml of tetrahydrofuran was stirred at room temperature for 10 minutes. To this suspension was added 3.137 g of t-butyldimethylsilyl chloride in one portion. The resulting mixture was stirred at room temperature for 8 hours and 10% aqueous sodium bicarbonate was added. After 5 minutes the mixture was filtered through Celite and washed with methylene chloride. The filtrate was washed with water and brine and dried. Removal of the solvent and flash chromatography of the residue on silica gel gave the desired silylether as an oil. $^1H$ NMR (200 MHz) 0.04 and 0.06 (2 s, $SiCH_3$), 0.89 [s, 9H, $SiC(CH_3)_3$], 1.18 [s, 9H, $C(CH_3)_3$], 2.60 (d, 1H, 3′-OH), 3.69-4.05 (br m, 9H), 4.16 (m, 2H), 4.53 (m, 1H), 4.55-4.75 (m, 8H, $CH_2OPh$), 4.85 (s, 2H, $OCH_2OBn$), 5.03 (s, 1H, $C_1'$-H), 7.31 (m, 20H, Ar-H).

Example 41

5′-O-[(1,1-Dimethylethyl)dimethylsilyl]-2′-O-[(phenylmethoxy)methyl]-β-D-ribofuranosyl-2,3,4-tris-O-(phenylmethyl)-D-ribitol

To a solution of 11.7 g of the ester from Example 40 in 60 ml of ether under nitrogen at room temperature, was added 30 ml of lithium triethylborohydride (1.0 M in tetrahydrofuran) over 5 minutes. After stirring 3 hours, the reaction mixture was cooled to 0° C and 10% aqueous sodium bicarbonate solution was carefully added dropwise. The mixture was then filtered through Celite and evaporated. The residue was dissolved in methylene chloride, washed with water and brine, and dried. After removal of the solvent and chromatography of the residue, the desired diol was obtained as a syrup. $^1$H NMR (200 MHz) 0.01 and 0.03 (2 s, $SiCH_3$), 0.89 [s, 9H, $SiC(CH_3)_3$], 2.23 (t, 1H, 5-OH), 2.55 (d, 1H, 3′-OH), 3.64-4.21 (br m, 12H), 4.50-4.71 (m, 8H, $CH_2OPh$), 4.85 (s, 2H, $OCH_2OBn$), 5.01 (s, 1H, $C_1$′-H), 7.22 (m, 20H, Ar-H).

Example 42

5′-O-[(1,1-Dimethylethyl)dimethylsilyl]-2′-O-[(phenylmethoxy)methyl]-β-D-ribofuranosyl-5-O-[(methoxy-phenyl)-diphenylmethyl]-2,3,4-tris-O-(phenylmethyl)-D-ribitol

To a solution of 6.69 g of the diol from Example 41, 2.06 ml of pyridine and 519 mg of dimethylaminopyridine in 100 ml of methylene chloride under nitrogen at room temperature was added 3.408 g of monomethoxytrityl chloride in 2 portions during 30 minutes. The resulting mixture was stirred at room temperature for 24 hours then methanol was added, followed in 5 minutes by 10% aqueous sodium bicarbonate solution. The reaction mixture was then diluted with methylene chloride and the organic phase was washed with 10% aqueous sodium bicarbonate, 5% copper sulfate solution and brine. The solution was dried, evaporated, and the residue was chromatographed on silica gel to afford the desired trityl ether as a yellow oil. $^1$H NMR (300 MHz) 0.01 and 0.03 (2 s, 6H, $SiCH_3$), 0.88 [s, 9H, $C(CH_3)_3$], 2.56 (d, 1H, OH), 3.56 (m, 2H), 3.68 (m, 3H), 3.76 (s, 3H, $OCH_3$), 3.82-3.87 (br m, 5H), 4.02 (br d, 1H, $C_2$′-H), 4.14 (br q, 1H, $C_3$′-H), 4.44-4.77 (br m, 8H, $CH_2OPh$), 4.82 (s, 2H, $OCH_2OBn$), 4.99 (s, 1H, $C_1$′-H), 6.74 (d, 2H, m-Ar-H on $PhOCH_3$), 7.09-7.56 (m, 32H, Ar-H).

Example 43

5′-O-[(1,1-Dimethylethyl)dimethylsilyl]-3′-O-(1,4-dioxopentyl)-2′-O-[(phenylmethoxy)methyl]-β-D-ribofuranosyl-2,3,4-tris-O-(phenylmethyl)-5-O-[(4-methoxyphenyl)-diphenylmethyl]-D-ribitol

To a septum-sealed flask containing 2.19 g of the alcohol from Example 42, 360 mg of levulinic acid and 379 mg of dimethylaminopyridine in 25 ml of ether, under argon at room temperature, was added a solution of 835 mg of dicyclohexylcarbodiimide in 20 ml of ether. The resulting mixture was stirred at room temperature for 24 hours and then filtered through Celite. The filtrate was concentrated and the residue was chromatographed to give the desired ester as a yellow syrup. $^1$H NMR (300 MHz) 0.00 and 0.02 (2 s, 6H), $SiCH_3$), 0.86 [s, 9H, $C(CH_3)_3$], 2.15 (s, 3H, $CH_3COCH_2CH_2$), 2.61 and 2.70 (2 br t, 4H, $CH_3COCH_2CH_2$), 3.54 (m, 2H), 3.63 (d d, 1H), 3.70 (m, 1H), 3.75 (s, 3H, $OCH_3$), 3.80-3.96 (br m, 3H), 4.14 (m, 1H, $C_4$′-H), 4.29 (d d, 1H, $C_2$′-H), 4.44-4.72 (m, 8H, $CH_2OPh$), 4.75 (d, 2H, $OCH_2OBn$), 5.00 (d, 1H, $C_1$′-H), 5.15 (t, 1H, $C_3$′-H), 6.73 (d, 2H, m-Ar-H on $PhOCH_3$), 7.09-7.45 (m, 32H, Ar-H).

Example 44

5′-O-[(1,1-Dimethylethyl)dimethylsilyl]-3′-O-(1,4-dioxopentyl)-2′-O-[(phenylmethoxy)methyl]-β-D-ribofuranosyl-2,3,4-tris-O-(phenylmethyl)-D-ribitol

To a solution of 2.40 g of the trityl ether from Example 43 in 10 ml of methylene chloride at room temperature, was added 75 ml of a 3% trichloroacetic acid in methylene chloride solution, dropwise. The resulting red solution was stirred for 15 minutes and treated with 10% aqueous sodium bicarbonate. The mixture was then partitioned between water and methylene chloride. The organic phase was washed with brine and dried over anhydrous sodium sulfate. After removal of solvent and chromatography of the residue, the desired alcohol was obtained as an oil. $^1H$ NMR (300 MHz) 0.03 and 0.05 (2 s, 6H, $SiCH_3$), 0.89 [s, 9H, $C(CH_3)_3$], 2.17 (s, 3H, $CH_3COCH_2CH_2$), 2.25 (t, 1H, OH), 2.63 and 2.72 (2 br t, 4H, $CH_3COCH_2CH_2$), 3.63-3.78 (m, 6H), 3.82 (d d, 1H), 3.89 (t, 1H), 3.95 (d d, 1H), 4.19 (d d, 1H, $C_4'$-H), 4.32 (d d, 1H, $C_2'$-H), 4.52-4.70 (m, 8H, $CH_2OPh$), 4.72 and 4.80 (2 d, $OCH_2OBn$), 5.07 (d, 1H, $C_1'$-H), 5.17 (t, 1H, $C_3$-H), 7.33 (m, 20H, Ar-H).

## Example 45

3′-O-[[Bis(1-methylethyl)amino]-2-cyanoethylphosphino]-2′,5′-bis-O-(phenylmethyl)-1′-O-β-D-ribofuranosyl-5-O-[-(4-methoxyphenyl)diphenylmethyl]-2,3,4-tris-O-(phenylmethyl)-D-ribitol

A solution of 440 mg of the product from Example 29 in 5 ml of tetrahydrofuran was added to a solution of 0.19 ml of 2-cyanoethyl-N,N-diisopropylchlorophosphoramidite and 0.31 ml of diisopropylethylamine in 10 ml of tetrahydrofuran. After the solution was stirred at room temperature for 18 hours, it was poured into a 50:50 mixture of ethyl acetate and brine. The layers were separated, the aqueous layer was washed with additional ethyl acetate, and the organic layers were combined. Drying, evaporation, and chromatography of the residue on silica gel produced the desired compound as a colorless oil. $^1H$ NMR (200 MHz; characteristic signals): 1.11 (d, 6H, $NC(CH_3)_2$), 1.12 (d, 6H, $NC(CH_3)_2$).

## Example 46

5′-O-[(1,1-Dimethylethyl)dimethylsilyl]-3′-O-[[bis(1-methylethyl)amino]methoxyphosphino]-2′-O-[(phenylmethoxy)methyl]-β-D-ribofuranosyl-5-O-[(4-methoxyphenyl)diphenylmethyl]-2,3,4-tris-O-(phenylmethyl)-D-ribitol

To a septum-sealed flask containing 0.80 g of N,N-diisopropylmethylphosphonamidic chloride and 1.28 ml of diisopropylethylamine in 15 ml of tetrahydrofuran under argon at room temperature, was added a solution of 1.90 g of the alcohol of Example 42 in 40 ml of tetrahydrofuran. The resulting cloudy solution was stirred at room temperature over-night, and then diluted with ethyl acetate. The mixture was washed with 15% aqueous sodium bicarbonate and brine. After drying, the solvent was removed at reduced pressure. The residue was purified on a short column of silica gel to give the phosphoramidite as a colorless syrup. $^{31}P$ NMR (121.42 MHz) 151.70, 152.00.

## Example 47

3′-O-[[Bis(1-methylethyl)amino]methoxyphosphino]-5′-O-[(4-methoxyphenyl)diphenylmethyl)-2′-O-[-

(phenylmethoxy) methyl]-β-D-ribofuranosyl-2,3,4-tris-O-(phenylmethyl)-5-O-(trimethylacetyl)-D-ribitol

A 150 mg portion of the alcohol from Example 37 in 0.85 ml of tetrahydrofuran was reacted with 0.07 ml of N,N-diisopropylmethylphosphoramidic chloride as described in Example 46 to produce the desired compound as a syrup. $^{31}P$ NMR (121.42 MHz): 149.98 and 150.37.

## Example 48

1-O-[3-O-[(2-Cyanoethoxy)hydroxyphosphinyl]-2,5-bis-O-(phenylmethyl)-β-D-ribofuranosyl]-5-O-[(4-methoxyphenyl)diphenylmethyl]-2,3,4-tris-O-(phenylmethyl-D-ribitol, P → 5-ester with 1-O-[3-O-(1,4-dioxopentyl)-2,5-bis-O-(phenylmethyl)-β-D-ribofuranosyl]-2,3,4-tris-O-(phenylmethyl)-D-ribitol

A solution of 237 mg of the product from Example 31 in 5 ml of dry acetonitrile was added to a solution of 378 mg of the product from Example 45 and 132 mg of freshly sublimed tetrazole in 1 ml of acetonitrile at room temperature. After stirring for 24 hours, the solution was treated with 0.1M iodine in 2:1 (v/v) tetrahydrofuran-water. The solvents were evaporated and the residue was extracted with methylene chloride. This solution was washed with 5% aqueous sodium bisulfite and brine, dried, and concentrated to the desired compound.

## Example 49

1-O-[5-O-[(1,1-Dimethylethyl)dimethylsilyl]-3-O-(hydroxymethoxyphosphinyl)-2-O-[(phenylmethoxy)methyl]-β-D-ribofuranosyl]-5-O-[(4-methoxyphenyl)diphenylmethyl]-2,3,4-tris-O-(phenylmethyl)-D-ribitol, P → 5-ester with 1-O-[5-O-[(1,1-dimethylethyl)dimethylsilyl]-3-O-(1,4-dioxopentyl)-2-O-[(phenylmethoxy)methyl]-β-D-ribofuranosyl]-2,3,4-tris-O-(phenylmethyl)-D-ribitol

To a septum-sealed flask containing 2.15 g of the phosphoramidite of Example 46 and 494 mg of tetrazole under argon at room temperature, was added a solution of 1.19 g of the alcohol from Example 44 in 30 ml of acetonitrile. The resulting mixture was stirred at room temperature for about 24 hours and then 1.6 ml of pyridine was introduced into the flask. The reaction mixture was then treated with iodine in tetrahydrofuran-water until the red color persisted. After 10 minutes, the red solution was poured into chloroform and this was washed with 5% aqueous sodium bisulfite and brine. The aqueous phase was back-extracted with chloroform. The combined organic phase was dried and the solvent was removed at reduced pressure. The crude product was purified on silica gel to give the desired dimer as two diastereomers. $^{31}P$ NMR (121.4 MHz): 1.849 and 2.108 ppm.

## Example 50

1-O-[5-O-[(1,1-dimethylethyl)dimethylsilyl]-3-O-(hydroxymethoxyphosphinyl)-2-O-[(phenylmethoxy)methyl]-β-D-ribofuranosyl]-5-O-[(4-methoxyphenyl)diphenylmethyl]-2,3,4-tris-O-(phenylmethyl)-D-ribitol, P → 5-ester with 1-O-[3-O-[(1,1-dimethylethyl)dimethylsilyl]-5-O-[(4-methoxyphenyl)diphenylmethyl]-2-O-[(phenylmethoxy)methyl]-β-D-ribofuranosyl]-2,3,4-tris-O-(phenylmethyl)-D-ribitol

A solution of 93 mg of the product from Example 39, 125 mg of the phosphoramidite from Example 47, and 37 mg of tetrazole in 2 ml of acetonitrile was reacted and oxidized as described in Example 49 to yield

the desired dimer as an oil. $^{31}P$ MNR (121.42 MHz): 0.166 and 0.257.

Example 51

1-O-[5-O-[(1,1-Dimethylethyl)dimethylsilyl]-3-O-(hydroxymethoxyphosphinyl)-2-O-[(phenylmethoxy)methyl]-β-D-ribofuranosyl]-2,3,4-tris-O-(phenylmethyl)-D-ribitol, P → 5-ester with 1-O-[5-O-[1,1-dimethylethyl)dimethylsilyl]-3-O-(1,4-dioxopentyl)-2-O-[(phenylmethoxy)methyl]-β-D-ribofuranosyl]-2,3,4-tris-O-(phenylmethyl)-D-ribitol

A 162 mg portion of the dimer from Example 49 was dissolved in 4 ml of 3% trichloroacetic acid in methylene chloride at room temperature. The solution was stirred for 10 minutes and the reaction was quenched with 10% aqueous sodium bicarbonate. The mixture was diluted with methylene chloride and then washed with 10% aqueous sodium bicarbonate and water. The organic phase was dried, evaporated, and chromatographed on silica gel to yield the desired alcohol as a colorless oil. $^{31}P$ NMR (121.4 MHz): 1.85 and 2.13.

Example 52

1-O-[5-O-[(1,1-dimethylethyl)dimethylsilyl]-3-O-(hydroxymethoxyphosphinyl)-2-O-[(phenylmethoxy)methyl]-β-D-ribofuranosyl]-5-O-[(4-methoxyphenyl)diphenylmethyl]-2,3,4-tris-O-(phenylmethyl)-D-ribitol, P → 5-ester with 1-O-[5-O-[1,1-dimethylethyl)dimethylsilyl]-2-O-[(phenoxymethoxy) methyl]-β-D-ribofuranosyl]-2,3,4-tris-O-(phenylmethyl) -D-ribitol

The dimer from Example 49 (800 mg) was dissolved in 3 ml of methylene chloride and 5 ml of 0.5M hydrazine in acetic acid-pyridine (1:4 v/v) was then added. The resulting yellow solution was stirred at room temperature for 15 minutes, then the reaction was quenched with 0.2 ml of acetyl acetone. The solution was poured into chloroform and washed with 10% aqueous sodium bicarbonate, water, 5% copper sulfate solution and brine. The combined aqueous phase was back-extracted with chloroform. After drying, the organic phase was evaporated and purified by chromatography on silica gel to give the desired alcohol as a yellow oil. $^{31}P$ NMR (121.4 MHz): 1.85, 2.09.

Example 53

1-O-[5-O-[(1,1-Dimethylethyl)dimethylsilyl]-3-O-(hydroxymethoxyphosphinyl)-2-O-[(phenylmethoxy)methyl]-β-D-ribofuranosyl]-5-O-[(4-methoxyphenyl)diphenylmethyl]-2,3,4-tris-O-(phenylmethyl)-D-ribitol, P → 5-ester with 1-O-[5-O-[1,1-dimethylethyl)dimethylsilyl]3-O-[[bis(1-methylethyl)amino]methoxyphosphino]-2-O-[(phenylmethoxy)-methyl]-β-D-ribofuranosyl]-2,3,4-tris-O-(phenylmethyl)-D-ribitol

The compound was prepared from the alcohol of Example 52 using the conditions detailed in Example 46 to yield a syrup. $^{31}P$ NMR (121.42 MHz): 0.013, 0.305, 149.97, and 150.23.

Example 54

1-O-[5-O-[(1,1-Dimethylethyl)dimethylsilyl]-3-O-(hydroxymethoxyphosphinyl)-2-O-[(phenylmethoxy)methyl]-β-D-ribofuranosyl]-5-O-[(4-methoxyphenyl)diphenylmethyl]-2,3,4-tris -O-(phenylmethyl)-D-ribitol, P → 5-ester with 1-O-[5-O-[(1,1-dimethylethyl)dimethylsilyl]-3-O-(hydroxymethoxyphosphinyl)-2-O-[(phenylmethoxy)methyl]-β-D-ribofuranosyl]-2,3,4-tris-O-(phenylmethyl)-D-ribitol, P → 5-ester with 1-O-[5-O-[(1,1-dimethylethyl)dimethylsilyl]-3-O-(hydroxymethoxyphosphinyl)-2-O-[(phenylmethoxy)methyl]-β-D-ribofuranosyl]-2,3,4-tris-O-(phenylmethyl)-D-ribitol, P → 5-ester with 1-O-[5-O-[(1,1-dimethylethyl)dimethylsilyl]-3-O-(1,4-dioxopentyl)-2-O-[(phenylmethoxy)-methyl]-β-D-ribofuranosyl]-2,3,4-tris-O-(phenylmethyl)-D-ribitol

A 95 mg portion of the phosphoramidite from Example 53, 65.2 mg of the alcohol from Example 51 and 13.1 mg of tetrazole in 1 ml of tetrahydrofuran were reacted and oxidized as described in Example 49 to yield the desired com pound as a mixture of enantiomers, epimeric at the phosphate esters. One of the enantiomers was isolated by chromatography on silica gel. $^{31}P$ NMR (121.42 MHz): 0.089, 0.334, 0.404.

## Example 55

1-O-[5-O-[(1,1-Dimethylethyl)dimethylsilyl]-3-O-(hydroxymethoxyphosphinyl)-2-O-[(phenylmethoxy)methyl]-β-D-ribofuranosyl]-2,3,4-tris-O-(phenylmethyl)-D-ribitol, P → 5-ester with 1-O-[5-O-[(1,1-dimethylethyl)dimethylsilyl]-2-O-[(phenylmethoxy)methyl]-β-D-ribofuranosyl]-2,3,4-tris-O-(phenylmethyl)-D-ribitol

A portion of the product of Example 51 was treated with hydrazine as described for the preparation of the product of Example 52 to yield the desired diol as a colorless oil. $^{31}P$ NMR (121.42 MHz): 0.221 and 0.473.

## Example 56

1-O-[5-O-[(1,1-Dimethylethyl)dimethylsilyl]-3-O-(dihydroxyphosphinyl)-2-O-[(phenylmethoxy)methyl]-β-D-ribofuranosyl]-2,3,4-tris-O-(phenylmethyl)-D-ribitol, P → 5-ester with 1-O-[5-O-[(1,1-dimethylethyl)dimethylsilyl]-2-O-[(phenylmethoxy)methyl]-β-D-ribofuranosyl]-2,3,4-tris-O-(phenylmethyl)-D-ribitol, (1,1-dimethylethyl) ammonium salt

A 60 mg sample of the product from Example 55 was dissolved in 10 ml of t-butylamine and the solution was heated at reflux for 24 hours. The t-butylamine was removed by blowing a stream of nitrogen over the reaction. The residue was applied to a column of silica gel, impurities were washed away, and the desired compound was obtained by washing the silica gel thoroughly with ethyl acetate and methanol. The combined eluents were evaporated to yield the compound as an oil. $^{31}P$ NMR (121.42 MHz): -0.654 ppm.

## Example 57

1-O-[3-O-(Dihydroxyphosphinyl)-β-D-ribofuranosyl]-D-ribitol, P → 5-ester with 1-O-(β-D-ribofuranosyl)-D-ribitol, sodium salt

The product from Example 56 was dissolved in 1.1 ml of 1.0M tetra-n-butylammonium fluoride and stirred for 7 hours at room temperature. The solvent was evaporated and the residue was re-dissolved in aqueous methanol. The catalyst, 10% palladium-on carbon, was added and the mixture was hydrogenated at 15 psi for 5 hours at room temperature. The reaction was filtered, evaporated, diluted with water, and extracted with ether and hexanes. The aqueous phase was stirred with Dowex 50W-X8 ($Na^+$ form) ion

exchange resin, then filtered through a column filled with the same resin, eluting with water. The water was evaporated and the residue was purified by chromatography on silica gel to yield the desired compound. $^{31}P$ NMR (121.42 MHz, $D_2O$): 1.136 ppm.

## Example 58

### 1′-β-D-Ribofuranosyl-1-D-ribitol

Application of the de-protection procedure described in Example 57 to the product of Example 41 yielded the desired compound. $[\alpha]_D^{23}$ = -35° (c 4.0, $H_2O$); $^1H$ NMR (300 MHz, $D_2O$) 3.58-3.72 (m, 4H), 3.74-3.84 (m, 4H), 3.89 (m, 1H), 4.00 (m, 1H, $C_4'$-H), 4.07 (d, 1H, $C_2'$-H0, 4.22 (dd, 1H, $C_3'$-H), 4.99 (s, 1H, $C_1'$-H); $^{13}C$ NMR (75.43 MHz, $D_2O$): 65.10, 65.19, 71.33, 72.95, 73.30, 74.67, 74.89, 77.06, 85.44, 109.59; MS (CI, glycerol, m/e, %) 377 ($M+gly+H^+$, 1.1), 285 ($M+H^+$, 3.8); HRMS (CI, glycerol, m/z) for $C_{10}H_{21}O_9$ - ($M+H^+$), calculated 285.1186, found 285.1187.

## Example 59

### 2$-O-[5-O-[(1,1-Dimethylethyl)dimethylsilyl]-3-O-(hydroxymethoxyphosphinyl)-2-O-[(phenylmethoxy)methyl]-β-D-ribofuranosyl]-5-O-[(4-methoxyphenyl)diphenylmethyl]-2,3,4-tris-O-(phenylmethyl)-D-ribitol, P → 5-ester with 1-O-[5-O-[(1,1-dimethylethyl)dimethylsilyl]-3-O-(hydroxymethoxyphosphinyl)-2-O-[(phenylmethoxy)methyl]-β-D-ribofuranosyl]-2,3,4-tris-O-(phenylmethyl)-D-ribitol, P → 5-ester with 1-O-[5-O-[(1,1-dimethylethyl)dimethylsilyl]-3-O-(hydroxymethoxyphosphinyl)-2-O-[(phenylmethoxy)methyl]-β-D-ribofuranosyl]-2,3,4-tris-O-(phenylmethyl)-D-ribitol, P → 5-ester with 1-O-[5-O-[(1,1-dimethylethyl)dimethylsilyl]-2-O-[(phenylmethoxy)methyl]-β-D-ribofuranosyl]-2,3,4-tris-O-(phenylmethyl)-D-ribitol

The product of Example 54 was treated with hydrazine as described in Example 52 to yield the desired compound.

## Example 60

### Benzyl 2,3-O-benzylidine-β-D-ribofuranoside

A flask containing 1 g of ribose, 1.415 g of benzaldehyde, and 2.884 g of benzyl alcohol was vigorously shaken while 0.2 ml of concentrated sulfuric acid was added dropwise. The resulting yellow solution was stirred at room temperature for 18 hours and heated at 80° C for 1 hour. It was diluted with ether and the ether phase was washed with 10% sodium bicarbonate solution, concentrated sodium hydrogen sulfate solution, and then water. The organic phase was dried, the solvent was evaporated, and the residue was chromatographed on silica gel to give the mixture of endo- and exo-ribofuranosides as colorless crystal. m.p. 46-50° C.

## Example 61

### Benzyl 5-O-[[bis(1-methylethyl)amino]methoxyphosphino]-2,3-O-(phenylmethylidine)-β-D-ribofuranoside

The product from Example 60 (800 mg), 1.7 ml of diisopropylethylamine and 1.16 ml of N,N-diisopropylmethylphosphoramidic chloride in 6 ml of tetrahydrofuran was reacted and purified as described in Example 46 to yield the desired phosphoramidite as an oil. $^{31}P$ NMR (121.42 MHz): 149.32 and 149.44 ppm.

Example 62

1-Dioxy-1-[[(phenylmethoxy)carbonyl]amino]-2,3,4-tris-O-(phenylmethyl)-D-ribitol

To a solution of the aldehyde from Example 10 (prior to sodium borohydride reduction) in 50 ml of absolute ethanol was added 2.159 g of sodium acetate and 177 mg of sodium cyanoborohydride. After 30 minutes of stirring at room temperature, the ethanol was evaporated and the residue was dissolved into chloroform. This solution was washed with brine, dried, and evaporated to yield the crude intermediate amine. The entire amount of amine was dissolved into 50 ml of methylene chloride and to the stirred solution was added 1.2 ml triethylamine followed by 0.64 ml of benzyl chloroformate. After 30 minutes, 3.5 g of trichloroacetic acid was added, and stirring was continued for 15 minutes. The organic solution was washed with aqueous sodium bicarbonate, dried and evaporated. Chromatography on silica gel yielded the desired compound as an oil. MS (Cl, $NH_3$) 556 ($M^+$ +1).

Example 63

1-O-[5-O-[(1,1-Dimethylethyl)dimethylsilyl]-3-O-(hydroxymethoxyphosphinyl)-2-O-[(phenylmethoxy)methyl]-β-D-ribofuranosyl]-5-O-[(4-methoxyphenyl)diphenylmethyl]-2,3,4-tris-O-(phenylmethyl)-D-ribitol, P → 5-ester with 1-O-[5-O-[(1,1-dimethylethyl)dimethylsilyl]-3-O-(hydroxymethylphosphinyl)-2-O-[(phenylmethoxy)methyl]-β-D-ribofuranosyl]-2,3,4-tris-O-(phenylmethyl)-D-ribitol, P → 5-ester with 1-O-[5-O-[(1,1-dimethylethyl)dimethylsilyl]-3-O-(hydroxymethoxyphosphinyl)-2-O-[(phenylmethoxy)methyl]-β-D-ribofuranosyl]-2,3,4-tris-O-(phenylmethyl)-D-ribitol, P → 5-ester with 1-O-[5-O-[(1,1-dimethylethyl)dimethylsilyl]-3-O-(hydroxymethoxyphosphinyl)-2-O-[(phenylmethoxy)methyl]-β-D-ribofuranosyl]-2,3,4-tris-O-(phenylmethyl)-D-ribitol, P → 5-ester with phenylmethyl 2,3-O-(phenylmethylene)-β-D-ribofuranoside

The product of Example 59 was reacted with the phosphoramidite described in Example 61 and oxidized to the desired compound as described in Example 49.

Example 64

1-O-[3-O-(Dihydroxyphosphinyl)-β-D-ribofuranosyl]-D-ribitol, P → 5-ester with 1-O-[3-O-(dihydroxyphosphinyl)-β-D-ribofuranosyl]-D-ribitol, P → 5-ester with 1-O-[3-O-hydroxymethoxyphosphinyl)-β-D-ribofuranosyl]-D-ribitol, P → 5-ester with 1-O-[3-O-(hydroxymethoxyphosphinyl)-β-D-ribofuranosyl]-D-ribitol, P → 5-ester with ribose

The product of Example 63 was fully de-protected using the following sequence: the methyl group was removed from the phosphate ester by refluxing the compound in t-butylamine as described in Example 55. The silyl protect ing groups were removed using tetra-n-butylammonium fluoride and the benzyl, benzylidine, and monomethoxytrityl groups were cleaved by catalytic hydrogenation in the presence of 10% palladium-on-charcoal as described in Example 57. The filtrate from the latter procedure was extracted with

ether and hexanes, and the aqueous phase was stirred with Dowex 50W-X8 ion exchange resin ($Na^+$ form). The filtrate was evaporated to yield the desired compound.

Macromolecular conjugates of the novel polysaccharides, such as polysaccharide of Example 62, are prepared by covalently reacting the polysaccharide with the macromolecule.

## Example 65

### Conjugation to an immunogenic protein

The product of Example 64 was dissolved in 0.4M potassium phosphate buffer, pH = 6.7, at a final concentration of 5mM. A solution of carrier protein in 1% β-D-octylglucoside at a concentration of 0.235 mM. To a 1 ml sample of the solution of the product of Example 64 in a screwtop plastic tube was added 1 ml of the carrier protein preparation, 100 µl of 10% β-D-octylglucoside, and 5 µmoles of sodium cyanoborohydride. The tube was sealed and the mixture was stirred for six days at 37° C. The reaction mixture was dialyzed against phosphate buffered saline containing 1% β-D-octylglucoside to yield the purified carrier protein-synthetic PRP conjugate.

The macromolecular conjugates of this invention will elicit poly(ribosyl-ribitolphosphate) antibody formation and induce active immunization in warm-blooded animals against infection caused by the pathogen Haemophilus influenzae type b as shown by the data in Table I. The conjugate vaccine (0.5 ml per dose) was injected subcutaneously into three week old Sprague-Dawley rats who had been acclimated for one week prior to once a week administration for three weeks.

TABLE I

| Antibody Response of Young Rats to PRP-Carrier Protein Conjugate | | | |
|---|---|---|---|
| Vaccine | Dose µg PRP/0.5 ml | GMT Antibody to PRP(ng/ml)* WEEKS POST ADMINISTRATION | |
| | | 4 | 5 |
| Phosphate Buffered Saline PBS (Control) | - | <40 | <40 |
| PRP | 10 | <40 | <40 |
| Connaught | 5 | <298 | 418 |
| PRP - Carrier Protein Conjugate | 5 | >292 | 635 |

*Five rats per group were injected with vaccine. The geometric-mean titer (GMT) of antibody was computed from the five discrete titers.

## Claims

1. A compound of the formula:

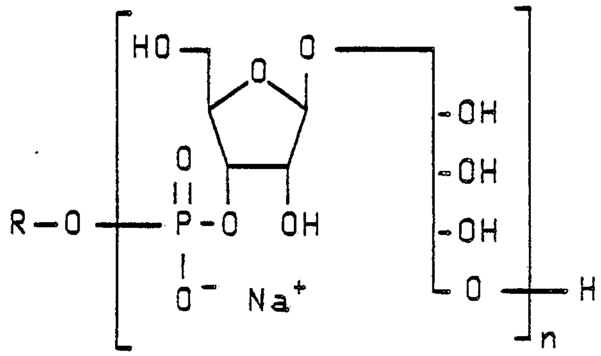

wherein n is an integer of from 2 to 30, and R is a spacer fragment defined by
$R'$-$(CHOH)_m$-$CH_2$-
wherein m is an integer of from 0 to 3; and $R'$ is CHO, COY, $CH_2X$, $CH_2NH_2$, or $CH_2SH$; Y is halo, N-hydroxysuccinimido, or hydrazino; and X is halogen, methanesulfonyl, trifluoromethanesulfonyl, or toluenesulfonyl.

2. A compound according to Claim 1 wherein n is 4, m is 3, and $R'$ is CHO.

3. A compound according to Claim 1 wherein n is 3, m is 3, and $R'$ is $CH_2NH_2$.

4. A compound according to Claim 1 wherein n is 3, m is 3, and $R'$ is $CH_2SH$.

5. A macromolecular conjugate of the formula:

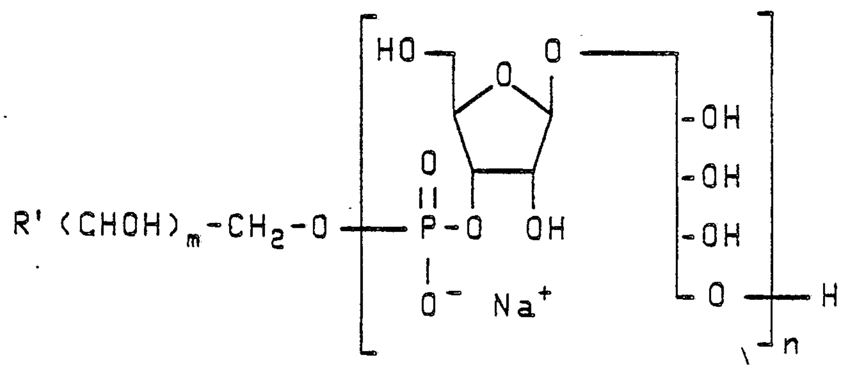

wherein n is an integer of from 2 to 30, m is an integer 0 to 3 and $R'$ is:
P-S-S-$CH_2$-

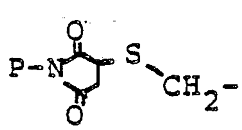

P-NHCO-
P-$NHCH_2$-
P-N=CH-
P-$CONHCH_2$-
P-$CH_2NHNHCO$-
P-CH=NNHCO-
P-$CH_2NHNHCO$-
and P is a macromolecule.

6. A compound according to Claim 5 wherein the macromolecule is a protein capable of inducing an immune response.

7. A compound according to Claim 6 wherein the protein is tetanus toxin, tetanus toxoid, diphtheria toxin or diphtheria toxoid.

8. A method of eliciting poly(ribosyl-ribitolphosphate) antibody formation and inducing immunization in warm-blooded animals against infection caused by the pathogen Haemophilus influenzae type b which comprises administering to the animal a pharmaceutically effective amount of a macromolecular conjugate of a compound of the formula:

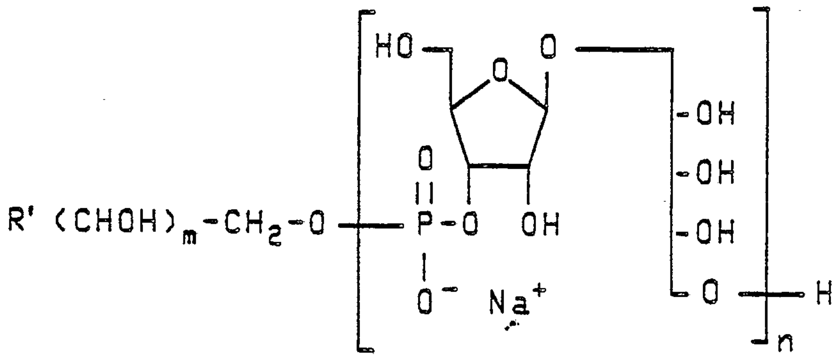

wherein n is an integer of from 2 to 30, m is an integer 0 to 3 and R′ is:
P-S-S-$CH_2$-

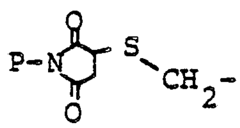

P-NHCO-
P-$NHCH_2$-
P-N=CH-
P-$CONHCH_2$-
P-$CH_2NHNHCO$-
P-CH=NNHCO-
P-$CH_2NHNHCO$-
and P is a macromolecule.

9. A vaccine against the pathogen Haemophilus influenzae type b comprising a macromolecular conjugate of the formula:

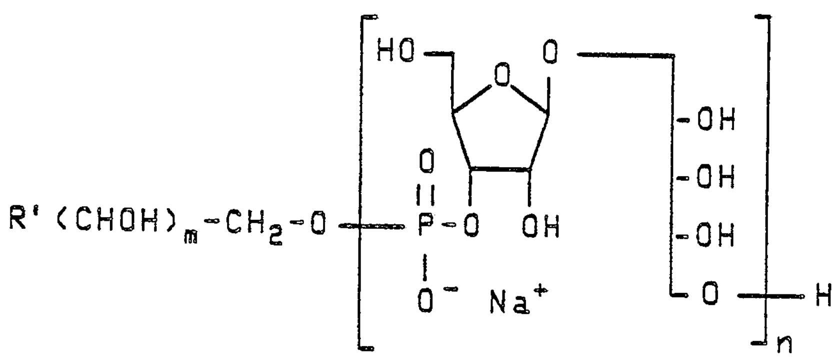

wherein n is an integer of from 2 to 30, m is an integer 0 to 3 and R′ is:
P-S-S-$CH_2$-

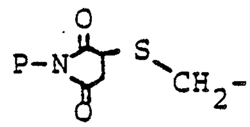

P-NHCO-
P-$NHCH_2$-
P-N = CH-
P-$CONHCH_2$-
P-$CH_2NHNHCO$-
P-CH = NNHCO-
P-$CH_2NHNHCO$-
P is a macromolecule, in a suitable carrier.

10. A process for preparing a compound of the formula:

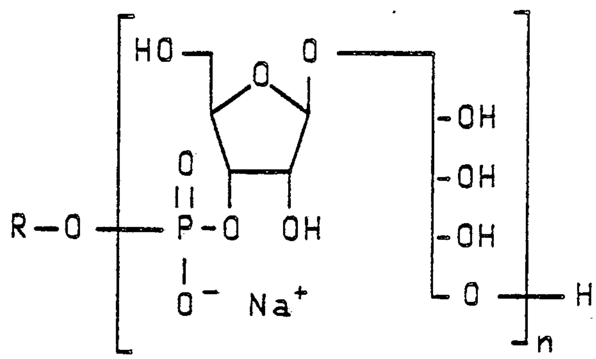

wherein n is an integer of from 2 to 30, and R is a spacer fragment defined by
$R'-(CHOH)_m-CH_2-$
wherein m is an integer of from 0 to 3; and R′ is CHO, COY, $CH_2X$, $CH_2NH_2$, or $CH_2SH$; Y is halo, N-hydroxysuccinimido, or hydrazino; and X is halogen, methanesulfonyl, trifluoromethanesulfonyl, or toluenesulfonyl comprising:

a) assembly of a ribitol unit from ribose such that the protecting groups on the $C_1$ and $C_5$ primary hydroxyl groups can be selectively and independently removed without concurrent removal of the protecting groups on the secondary hydroxyl groups of the ribitol or any protecting groups from the ribose unit;

b) assembly of a ribose unit containing activating functionality at its anomeric carbon and protecting functionality on its remaining hydroxyl groups such that the protecting group at $C_3$ can be removed independently of the protecting groups on the hydroxyls at $C_2$ and $C_5$ of the ribose unit or any of the protecting groups on the ribitol unit;

c) reaction of an appropriately protected ribitol unit with an appropriately protected and activated ribose unit to generate a ribosyl-ribitol monomer linked through $C_1$ of the ribose and $C_1$ of the ribitol to produce substantially the $\beta$-configuration at the juncture of the two units;

d) removing the protecting group at $C_3$ of the terminal ribose unit while leaving all other protecting groups intact;

e) reacting the $C_3$ hydroxyl group with an appropriate phosphoramidic chloride to generate a phosphoramidite intermediate;

f) selectively removing the protecting group from the primary hydroxyl of the terminal ribose from step c) and reacting this with the phosphoramidite intermediate from step e), followed by oxidation to a phosphotriester;

g) repeating steps d) to f) until the desired number of repeating units n have been assembled;

h) repeating steps d) and e) for introducing a phosphoramidite at $C_3$ of the terminal ribose, then introducing the spacer moiety R, containing the desired functional group R′, through the phosphoramidite at $C_3$ of the terminal ribose, followed by oxidation to a phosphotriester;

i) removal of all protecting groups and introducing sodium as the counterions of the phosphate groups.

11. A process for preparing a compound of the formula:

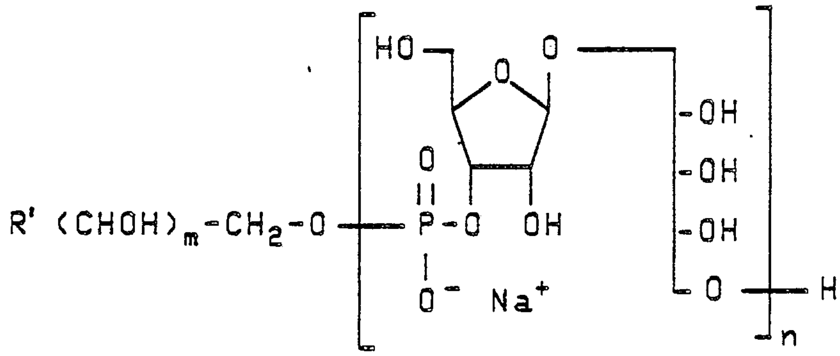

wherein n is an integer of from 2 to 30, m is an integer 0 to 3 and R′ is:
P-S-S-$CH_2$-

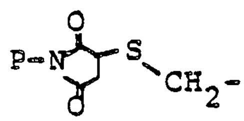

P-NHCO-
P-$NHCH_2$-
P-N=CH-
P-$CONHCH_2$-
P-$CH_2NHNHCO$-
P-CH=NNHCO-
P-$CH_2NHNHCO$-
and P is a macromolecule which comprises reacting a compound of the formula:

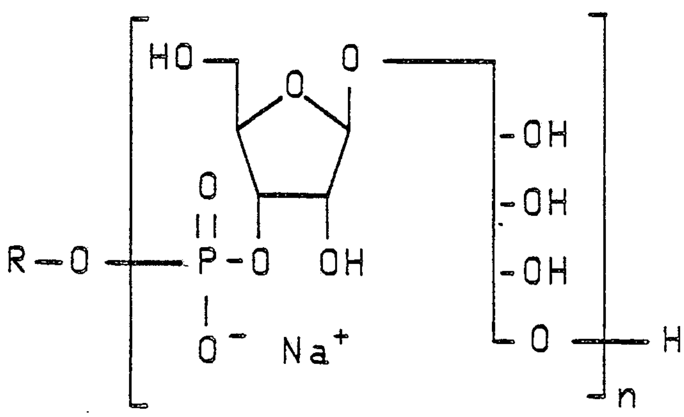

wherein n is an integer of from 2 to 30, and R is a spacer fragment defined by
R′-$(CHOH)_m$-$CH_2$-
wherein m is an integer of from 0 to 3; and R′ is CHO, COY, $CH_2X$, $CH_2NH_2$, or $CH_2SH$; Y is halo, N-hydroxysuccinimido, or hydrazino; and X is halogen, methanesulfonyl, trifluoromethanesulfonyl, or toluenesulfonyl with an immunogenic macromolecule.